# EUROPEAN PATENT SPECIFICATION

(11) **EP 0 777 452 B1**
(45) Date of publication and mention of the grant of the patent: **09.05.2001**
(21) Application number: 95930884.2
(22) Date of filing: 21.08.1995
(51) Int. Cl.: A61F 13/26

(54) **TAMPON APPLICATOR HAVING A SEMI-SPHERICALLY SHAPED PLEATED TIP**
TAMPONAPPLIKATOR MIT EINER HALBKUGELFÖRMIG GEFORMTEN GEFALTETEN SPITZE
APPLICATEUR DE TAMPON A BOUT PLISSE ET HEMISPHERIQUE

(30) Priority: 22.08.1994 US 294230; 06.09.1994 US 300987
(43) Date of publication of application: 11.06.1997
(73) Proprietor: KIMBERLY-CLARK WORLDWIDE, INC., Neenah, Wisconsin 54956 (US)
(72) Inventor: NIELSEN, Steven, James, Greenville, WI 54942 (US); KRUEGER, Allan, James, Winneconne, WI 54986 (US); RASMUSSEN, Noel, John, Oshkosh, WI 54901 (US); RENTMEESTER, Tammy, Jo, Appleton, WI 54915 (US); TEWS, Richard, Roy, Larsen, WI 54947 (US); WEYENBERG, Jeffrey, Michael, Appleton, WI 54915 (US)
(74) Representative: DIEHL GLAESER HILTL & PARTNER
(86) International application number: US9510635
(87) International publication number: WO9605793

(56) References cited:
- US-A- 3 204 635
- US-A- 3 433 225
- US-A- 3 753 437

## Description

### FIELD OF THE INVENTION

This invention relates to a tampon applicator having a semi-spherically shaped pleated tip for facilitating insertion of a catamenial tampon into a body cavity. More specifically, this invention relates to an apparatus and method for crimping, pleating and forming a tip on a hollow tube.

### BACKGROUND OF THE INVENTION

Catamenial tampons and other types of absorptive media are routinely inserted into body cavities, such as a woman's vagina, to absorb menstrual fluid, blood and other kinds of body fluid. One convenient way to position such absorbent tampons into a body cavity is through the use of an applicator. Comfortable and clean insertion of the absorbent tampon are keys to repeated sale of such applicators. In addition, the applicator should be capable of inserting the absorbent tampon into the body cavity using an acceptable amount of expulsion force.

Tampon applicators are available in a variety of shapes and sizes with the two piece telescopically assembled design being the most prevalent. In the two piece applicator, the tampon is housed in an outer tube and is expelled into a woman's vagina by an inner member which is telescopically mounted in the outer tube and acts as a plunger. Some tampon applicators utilize a hollow tube having an open insertion end through which the tampon is always exposed while other applicators utilize a completely closed or partially closed design. A thin film membrane can cover the insertion end of an applicator to completely enclose the forward end of a tampon while folds and pleats can be used to partially enclose the forward end of a tampon and protect it from contamination. Still other applicators, especially plastic applicators, have a plurality of flexible petals formed on the forward end of the outer tube which can flex radially outward to allow the tampon to be expelled. It will be appreciated that the diameter of the applicator, the material from which it is formed, the basic configuration of the applicator, the size and shape of the tampon positioned in the applicator, as well as the ease of opening the forward end of the applicator will all influence the force required to expel the tampon therefrom. The expulsion force should be kept reasonably low to permit proper functioning of the applicator.

US-A-3 204635 discloses a catamenial tampon device which incorporates an outer, generally cylindrical tube having a smooth, generally conically shaped forward end. A tampon is disposed in a tampon ejecting means.

While many have tried to design and manufacture tampon applicators having these improved qualities, there still remains a need for a tampon applicator which is more comfortable to use. Those applicators having an open forward end tend to expose the dry absorbent fibers of the tampon to the interior walls of a woman's vagina and this can cause irritation during insertion. Commercially available plastic applicators, using a plurality of petal tips, separated by slots, can sometimes pinch or cut the vaginal tissue of a woman during insertion and cause discomfort. Paper applicators having partially or fully closed tips tend to require an increased expulsion force to expel the tampon from the applicator and this can cause the applicator to deform or cause the tampon to be inserted incorrectly. Such insertion can cause discomfort to the user.

Now a paper tampon applicator has been invented having a semi-spherically shaped pleated tip for facilitating comfortable insertion of an absorbent tampon into a woman's vagina while having a low expulsion force. In addition, an apparatus and method for crimping, pleating and forming an insertion tip on the end of a paper tampon applicator has been invented which provides a central aperture formed through the insertion tip. The central aperture allows the pleats to open with a minimum amount of expulsion force and provides a visual means for the consumer to verify that the applicator does contain an absorbent tampon.

### SUMMARY OF THE INVENTION

Briefly, this invention relates to a paper tampon applicator having a semi-spherically shaped pleated tip for facilitating insertion of a catamenial tampon into a woman's vagina. The tampon applicator includes a first member capable of housing an absorbent tampon. The first member has a central longitudinal axis and first and second ends. An insertion tip is integrally formed on the first end of the first member and extends outwardly therefrom. The insertion tip contains a small central aperture which extends therethrough and the aperture has a side wall which is aligned essentially parallel to the central longitudinal axis of the first member. The insertion tip contains a plurality of pleats arranged in a semi-spherical configuration. The pleats are capable of expanding radially outward as the tampon is expelled from the first member. The tampon applicator further includes a second member telescopically mounted in the second end of the first member. The second member is adapted to expel the tampon through the insertion tip as it is pushed into the first member.

The tampon applicator is also disclosed in combination with a catamenial tampon having a shaped nose which approximates the interior surface of the first member.

The invention also relates to an apparatus and method for crimping, pleating and forming a tip on a hollow tube. The apparatus includes a first punch having a tubular section sized to receive the tube and having a configured tip with a plurality of elongated grooves formed therein. The first punch also contains a shoulder formed at an opposite end of the tubular section which acts as a stop for the tube. The first punch and tube are mateable with a first die. The first die includes a base having a plurality of blades extending axially outward therefrom. Each blade is designed to engage with one of the grooves formed on the first punch and causes the tip of the tube to be crimped therebetween. After an end of the tube has been crimped, it is transformed into a plurality of pleats and configured into a semi-spherical configuration having a central aperture formed therethrough. This is accomplished using a second punch having a tubular section sized to receive the tube. The second punch also has a semi-spherically shaped tip with a pin extending outward from the apex thereof and a shoulder formed at an opposite end of the tubular section which acts as a stop for the tube. The second punch and tube are mateable with a second die. The second die includes a base having a semi-spherical cavity formed therein with a central opening formed at the bottom of the cavity. The cavity is sized to receive the second punch and the tube, and the opening is sized to receive only the pin. The method of engaging the punches and dies to crimp, pleat and form one end of the tube is also described.

The general object of this invention is to provide a paper tampon applicator having a semi-spherically shaped pleated tip for facilitating insertion of a catamenial tampon into a body cavity. A more specific object of this invention is to provide an apparatus and method for crimping, pleating and forming an insertion tip on an end of a paper tampon applicator.

Another object of this invention is to provide a tampon applicator having a uniquely formed tip which prevents premature contamination yet substantially encloses the forward end of an absorbent tampon.

Still another object of this invention is to provide a tampon applicator having a pleated tip which essentially encloses the forward end of an absorbent tampon and which can be opened with a minimum amount of force.

A further object of this invention is to provide an apparatus for crimping, pleating and forming the insertion end of a paper tampon applicator into a semi-spherical configuration having a central aperture formed therethrough.

A further object of this invention is to provide a paper tampon applicator which is economical to manufacture and easy to use.

Still another object of this invention is to provide a paper tampon applicator which will minimize discomfort to a woman when she inserts an absorbent tampon into her vagina.

Still another object of this invention is to provide an apparatus which is simple to build and easy to operate.

Still another object of this invention is to provide a method for crimping, pleating and forming the insertion end of a tampon applicator at high speeds.

Still further, an object of this invention is to provide a simple and economical method of crimping, pleating and forming the insertion end of a tampon applicator.

Still further, an object of this invention is to provide a paper tampon applicator which will minimize discomfort to a woman when she inserts an absorbent tampon into her vagina.

Still further, an object of this invention is to provide a spirally wound, convolutely wound or longitudinally seamed paper tampon applicator with an improved tip for facilitating insertion of an absorbent tampon into a woman's vagina.

Other objects and advantages of the present invention will become more apparent to those skilled in the art in view of the following description and the accompanying drawings.

### BRIEF DESCRIPTION OF THE DRAWINGS

Fig. 1 is a perspective view of a two piece, spirally wound paper tampon applicator.

Fig. 2 is a cross-sectional view of the tampon applicator shown in Fig. 1.

Fig. 3 is a left end view of the tampon applicator shown in Fig. 1 depicting eight pleats.

Fig. 4 is a cross-sectional view of the insertion tip taken along line 4--4 of Fig. 3 showing an aperture formed through the insertion tip and the aperture having a side wall aligned essentially parallel to the central longitudinal axis of the first member.

Fig. 5 is a cross-sectional view of an insertion tip integrally formed on the first member and having an aperture formed therethrough wherein the side wall of the aperture is aligned at an angle to the central longitudinal axis of the first member. Figure 5 is not according to the invention.

Fig. 6 is an alternative end view of a tampon applicator depicting three pleats.

Fig. 7 is still another alternative end view of a tampon applicator depicting sixteen pleats.

Fig. 8 is a schematic view of a pleat taken along line 8--8 of Fig. 3 depicting the shape and thickness of a pleat.

Fig. 9 is a cross-sectional view of the insertion tip taken along line 9--9 of Fig. 3 depicting one end of the pleats extending into the first member.

Fig. 10 is a cross-sectional view of an alternative embodiment of the insertion tip showing one end of the pleats terminating at a point where the insertion tip integrally joins the first member.

Fig. 11 is a cross-sectional view of another embodiment of the insertion tip showing one end of the pleats terminating at a point on the exterior surface of the insertion tip.

Fig. 12 is a perspective view of the tampon applicator showing the pleats in an open arrangement.

Fig. 13 is a perspective view of a paper tampon applicator having an inner tube and an outer tube.

Fig. 14 is a cross-sectional view of the tampon applicator shown in Fig. 13.

Fig. 15 is a left end view of the tampon applicator shown in Fig. 13 depicting eight pleats.

Fig. 16 is a schematic view of a pleat taken along line 16--16 of Fig. 15 depicting the shape and thickness of a pleat.

Fig. 17 is a side elevational view of the outer tube before the insertion tip is formed.

Fig. 18 is a right end view of the outer tube shown in Fig. 17.

Fig. 19 is a perspective view of a first punch.

Fig. 20 is a perspective view of a first punch having a plurality of grooves formed in the tip and showing the first punch being mateable with a first die.

Fig. 21 is a partial section view showing the first punch and outer tube mating with the first die.

Fig. 22 is a side elevational view of the outer tube showing the insertion tip after undergoing crimping.

Fig. 23 is a right end view of the outer tube shown in Fig. 22.

Fig. 24 is a perspective view of the second punch and the second die with the outer tube shown in phantom.

Fig. 25 is a partial section view showing the second punch and outer tube mating with the second die.

Fig. 26 is a side elevational view of the outer tube showing the insertion tip after undergoing crimping, pleating and forming into a semi-spherical configuration.

Fig. 27 is a right end view of the outer tube shown in Fig. 26.

Fig. 28 is a perspective view of the tampon applicator showing the pleats in an open arrangement after the tampon has been expelled by the inner tube.

### DETAILED DESCRIPTION OF THE PREFERRED EMBODIMENTS

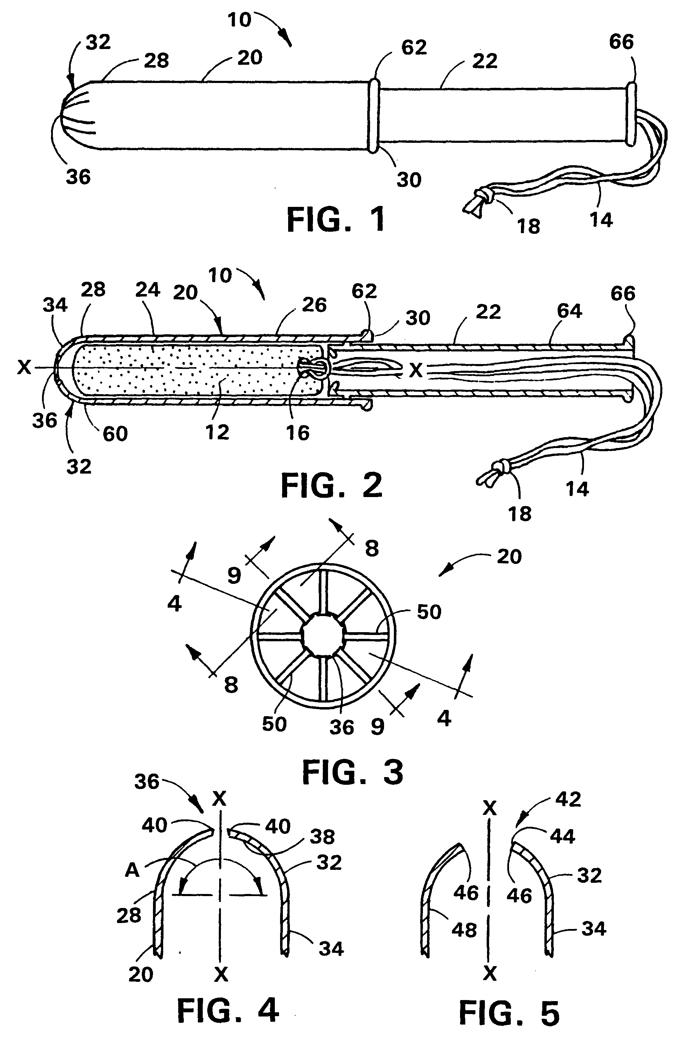

Referring to Figs. 1-3, a tampon applicator 10 is shown which is designed to house a catamenial tampon 12 and provide a comfortable means of inserting the tampon 12 into a woman's vagina. A tampon is an absorbent member primarily designed to be worn by a woman during her menstrual period to absorb menses, blood and other body fluid. The tampon 12 can be made from natural or synthetic fibers including cellulose fibers such as cotton or rayon, or artificial fibers such as polyester, polypropylene, nylon or blends thereof. Other types of fibers may also be used, such as cellulose sponge or a sponge formed from elastomeric materials. A blend of cotton and rayon fibers works well.

The tampon 12 is normally compressed into the form of a cylinder and can have a blunt, rounded or shaped forward end. The tampon 12 commonly has a withdrawal string 14 fastened to an end thereof which serves as a means for withdrawing the soiled tampon from the woman's vagina. The withdrawal string 14 can be looped through an aperture 16 formed transversely through the tampon 12. In addition, the withdrawal string 14 can have a knot 18 formed at it's free end to assure that the string 14 will not separate from the tampon 12.

The tampon applicator 10 includes a first member 20 and a second member 22. The first member 20 is preferably in the form of a spirally wound, convolutely wound or longitudinally seamed hollow tube which is formed from paper, paperboard, cardboard or a combination thereof. The first member 20, also commonly referred to as an outer tube, is fairly rigid and has a relatively small diameter of about 10 mm to about 20 mm. The first member 20 has a wall 24 with a predetermined thickness of about .2 mm to about .6 mm. The wall 24 can be constructed from a single ply of material or be formed from two or more plies which are bonded together to form a laminate. The use of two or more plies or layers is preferred for it enables the manufacture to use certain material in the various layers which can enhance the performance of the tampon applicator 10. When two or more plies are utilized, all the plies can be spirally wound, convolutely wound or longitudinally seamed to form an elongated cylinder. The wall 24 can be constructed using a smooth thin ply of material on the outside or exterior surface 26 which surrounds a coarser and possibly thicker ply. When the wall 24 contains at least three plies, the middle ply can be the thicker ply and the interior and exterior plies can be smooth and/or slippery to facilitate expulsion of the tampon 12 and to facilitate insertion of the first member 20 into a woman's vagina, respectively. By sandwiching a thick, coarser ply of material between two thin, smooth plies, an inexpensive first member 20 can be provided which is very functional. The wall 24 should contain one to four plies, although more plies can be utilized if desired.

The plies forming the wall 24 can be held together by an adhesive, such as glue, or by heat, pressure, ultrasonics, etc. The adhesive can be either water-soluble or water-insoluble. A water-soluble adhesive is preferred for environmental reasons in that the wall 24 will quickly break apart when it is immersed in water. Such immersion will occur should the first member 20 be disposed of by flushing it down a toilet. Exposure of the first member 20 to a municipal's waste treatment plant wherein soaking in water, interaction with chemicals and agitation all occur, will cause the wall 24 to break apart and evenly disperse in a relatively short period of time.

The inside diameter of the first member 20 is usually less than about .75 inches (about 19 mm) and preferably less than about .625 inches (about 16 mm). Although the exterior diameter of tampons do vary, most tampons utilized by women have an external diameter of less than about .75 inches (about 19 mm). However, if one desired to use this invention to administer medication to an animal, such as a farm animal, larger size tampons 12 could be used.

It should be noted that the first member 20 can be spirally wound, convolutely wound or longitudinally seamed into a cylindrical tubular shape. Alternatively, the material can be overlapped into a tubular configuration. Spirally or convolutely winding the first member 20 into a cylindrical tube is especially advantageous when the first member 20 is formed from a laminate. The reason for this is that when a laminate is circumferentially wound into a tube and a butt seam or an overlap is formed, the butt seam or the overlap can interfere with the later formation of pleats on the forward end thereof. A common problem with a rigid or stiff walled, tubular member having a relatively small diameter and a butt seam is that the seam has a tendency to come apart after formation if exposed to certain stress forces and/or high humidity. A problem with a tubular member having an overlap is that a small portion of the wall will be thicker than the remaining portion and this will cause problems when one tries to pleat one end of the tube. Accordingly, the first member 20 should preferably be formed into a cylindrical configuration without the presence of a butt seam or an overlap.

The first member 20 is sized and configured to house the absorbent tampon 12. As stated above, the first member 20 should have a substantially smooth exterior surface 26 which will facilitate insertion of the first member 20 into a woman's vagina. When the exterior surface 26 is smooth and/or slippery, the first member 20 will easily slide into a woman's vagina without subjecting the internal tissues of the vagina to abrasion. The first member 20 can be coated to give it a high slip characteristic. Wax, polyethylene, a combination of wax and polyethylene, cellophane and clay are representative coatings that can be applied to the first member 20 to facilitate comfortable insertion.

The first member 20 can be a straight, elongated cylindrical tube formed on a central longitudinal axis X--X. It is also possible to form the first member 20 into an arcuate shape. The arcuate or curved shape can assist in providing comfort when inserting the first member 20 into a woman's vagina. With a curved tampon applicator, it is possible to employ a curved tampon which again may be more comfortable for some women to use since the shape of the tampon may better fit the curvature of a woman's vagina.

The first member 20 has first and second spaced apart ends 28 and 30, respectively. The first member 20 can also have either a constant outer diameter or a stepped outer profile. Preferably, the first member 20 will have an essentially constant diameter over a major portion of it's length. Integrally formed on the first end 28 of the first member 20 and extending outwardly therefrom is an insertion tip 32. The insertion tip 32 is designed to facilitate insertion of the first member 20 into a woman's vagina in a comfortable manner. The insertion tip 32 is semi-spherical in configuration and has a diameter which is approximately equal to the outside diameter of the first member 20. The insertion tip 32 has a wall 34 with a thickness which is approximately equal to the thickness of the wall 24 which forms the first member 20. However, it is possible to construct the wall 34 so that it has a thickness which is less than or greater than the thickness of the wall 24, if desired.

Referring to Fig. 4, the insertion tip 32 is shown in cross-section with the semi-spherical configuration extending outward away from the first end 28 of the first member 20. The cross-section of the semi-spherical configuration spans an arc (A) of approximately 180 degrees. The semi-spherical configuration is formed on a diameter which is sized to be equal to or slightly smaller than the diameter of the first member 20. For example, if the outside diameter of the first member 20 is .64 inches (16.2 mm), the insertion tip 32 can be formed on a radius of about .32 inches (about 8.1 mm).

A relatively small aperture 36 is formed in the center of the semi-spherical or dome shaped insertion tip 32 and is coaxially aligned with the longitudinal axis X--X. The aperture 36 can have a diameter of at least about 1.5 mm, preferably between about 1.5 to about 5.0 mm, and more preferably, between about 3.0 to about 3.5 mm. Another way of sizing the diameter of the aperture 40 is to make it less than about 30% of the diameter of the first member 20, preferably, between about 10% to about 30% of the diameter of the first member 20, and most preferably, less than about 20% of the diameter of the first member 20. It should be noted that although the aperture 36 is described as a circle, it is possible to form the aperture 36 in other shapes such as a polygon, a square, a pentagon, a hexagon, an octagon, etc. The small aperture 36 should extend through the insertion tip 32 and have a side wall 38 which is aligned essentially parallel to the longitudinal axis X--X. In addition, the aperture 36 can be rounded or contain a radius 40 on it's exterior surface to assure that no sharp edges are present which could pinch or cut the sensitive tissues of a woman's vagina. The purpose of the small aperture 36 in the end of the insertion tip 32 is to facilitate the subsequent unfolding of the pleats during use, as will be described below. The aperture 36 also assures that the pleats will symmetrically open about the longitudinal axis X--X of the first member 20. A further benefit of the aperture 36 is that it provides a visual means for the user to inspect the tampon applicator 10 and assure herself that a tampon 12 is present in the first member 20.

The design in Fig. 4 is to be contrasted to the embodiment shown in Fig. 5, which is not an embodiment according to the invention. In Fig. 5 an enlarged aperture 42 is depicted having a side wall 44 which tapers downward and inward to from a point 46 adjacent to an interior surface 48 of the insertion tip 32. The sharp point 46 is more likely to pinch or trap vaginal tissue and therefore could cause discomfort during insertion. In addition, the larger diameter of the aperture 42 exposes a greater area of the absorbent tampon 12 and this could cause abrasion with the vaginal tissues during insertion. The embodiment shown in Fig. 4 is more desirable for comfort.

Referring again to Fig. 4, the configuration of the aperture 36 is preferred for it is smaller in diameter and therefore exposes a smaller amount of the absorbent tampon 12. Since a tampon is normally dry and consists of a plurality of absorbent fibers, it can cause abrasion against the walls of a woman's vagina as it is being inserted. By reducing the amount of surface area of the tampon 12 which is exposed to the vaginal tissue, one can decrease the discomfort during the insertion process. In addition, since the insertion tip 32 is almost closed, it also lowers the frictional force between the exterior surface 26 of the tampon applicator 10 and the walls of the vagina. Furthermore, the small diameter of the aperture 36 also decreases the possibility of trapping or pinching vaginal tissue therein.

Referring to Figs. 3, 6 and 7, the insertion tip 32 is shown having a plurality of pleats 50 which can radially open such that the insertion tip 32 has a diameter approximately equal to or greater than the diameter of the first member 20. Either an even or an odd number of pleats 50 can be present and the pleats 50 can be equally spaced apart or they can be non-uniformly arranged. Uniformly arranged pleats 50 are preferred but randomly arranged pleats 50 will work. For ease of manufacturing, it is preferred that the pleats 50 be equally spaced relative to one another. Each pleat 50 is a fold formed by doubling the material upon itself and then pressing or adhering the material into place. Although eight equally spaced apart pleats 50 are shown in Fig. 3, it is possible to utilize various numbers of pleats 50. The number of pleats 50 can vary from between three to about thirty-two pleats, preferably between about 5 to about sixteen pleats, and most preferably, between about 6 to about 12 pleats.

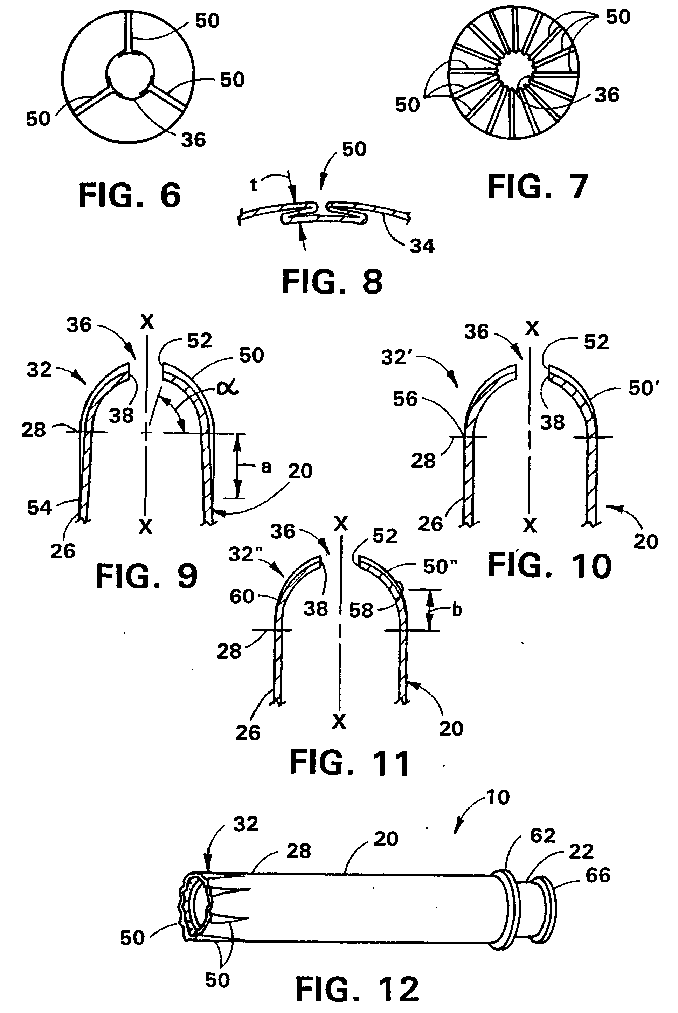

In Fig. 6, an embodiment is shown with three equally spaced pleats 50, while in Fig. 7, sixteen pleats 50 are displayed. The minimum number of pleats 50 should be no less than three because the force required to open the insertion tip 32 normally increases as the number of pleats 50 decrease. If the force becomes too large, the tampon applicator 10 could bend or deform during the insertion process and this may cause discomfort. When more than thirty-two pleats 50 are used, the expulsion force may be lowered but it becomes difficult to form so many pleats on the insertion tip 32.

Referring to Fig. 8, a schematic view of a pleat 50 is shown. The pleat 50 is obtained by folding the paper, paperboard, or cardboard material upon itself so that when each pleat 50 is opened or unfolded it will occupy a much larger surface area. The thickness of the material forming the insertion tip 32 can be equal to or slightly less than the thickness of the first member 20. For the first member 20, a thickness of about .1 mm to about .7 mm works fine. The insertion tip 32 can have a thickness between about .1 mm to about .5 mm. In the folded condition, the pleat 50 has a thickness, indicated by the letter "t" of less than about 0.7 mm, preferably between about .25 mm to about .35 mm. Another way of stating this is to say that the thickness of each pleat 50 in the folded condition will be greater than twice the thickness of the material from which the insertion tip 32 is constructed.

Referring to Figs. 9-11, three different embodiments of a pleat are depicted. In Fig. 9, the pleat 50 is depicted as having a first end 52 which coincides with the side wall 38 of the aperture 36. In other words, the first end 52 of the pleat 50 forms a portion of the arc of the aperture 36. The pleat 50 also has a second end 54 which coincides with a point located on the exterior surface 26 of the first member 20. This point is spaced a distance "a" from the location where the insertion tip 32 is integrally joined to the first member 20. By forming the pleat 50 with this particular length, one can control the amount of force needed to open the insertion tip 32 and push the tampon 12 therethrough. Usually, a lower force is required to open the pleats when each pleat 50 has a length which extends into the outer circumference of the first member 20.

In Fig. 9, the semi-spherical tip 32 spans a radial arc, identified as angle alpha (α), which extends from the first end 52 to the point where the semi-spherical shaped tip 32 is integrally joined to the first member 20. The angle alpha (α) is between about 60° to about 90°, preferably between about 75° to about 90°, and most preferably, greater than 80°. The angle alpha (α) would be 90° if the aperture 36 was not present. The size of the aperture 36 will partially determine the exact angle of the insertion tip 32. The angle alpha (α) should be as close to 90° as possible without completely enclosing the forward end of the tampon 12.

In Fig. 10, an alternative embodiment of an insertion tip 32' is depicted wherein a pleat 50' is shown having a first end 52 which coincides with the side wall 38 of the aperture 36. In other words, the first end 52 of the pleat 50' forms a portion of the arc of the aperture 36. The pleat 50' also has a second end 56 which coincides with the point where the insertion tip 32 is integrally joined to the first end 28 of the first member 20. By forming the pleat 50' with this particular length, one can control the amount of force needed to open the insertion tip 32 and push the tampon 12 therethrough. Although the force required to open the pleats 50' may be slightly greater than the force required with the design shown in Fig. 9, the force is still within acceptable limits.

In Fig. 11, a third embodiment of an insertion tip 32'' is depicted wherein a pleat 50" is shown having a first end 52 which coincides with the side wall 38 of the aperture 36. In other words, the first end 52 of the pleat 50" forms a portion of the arc of the aperture 36. The pleat 50" also has a second end 58 which coincides with a point located on an exterior surface 60 of the insertion tip 32. This point is spaced a distance "b" from the location where the insertion tip 32 is integrally joined to the first end 28 of the first member 20. By forming the pleat 50" with this particular length, one can control the amount of force needed to open the insertion tip 32 and push the tampon 12 therethrough. Although the force required to open the pleats 50" may be greater than the force required with the designs shown in Figs. 9 and 10, the force is still within acceptable limits.

It should be noted that both the length and diameter of commercially available tampons do vary and therefore the tampon applicators 10 should be manufactured in a variety of sizes. Tampons can vary in length from about 1 to about 3 inches (about 25.4 mm to about 76.2 mm) but preferably are about 2 inches (about 50.8 mm) in length. The tampon diameter will also vary from about .25 inches to about .75 inches (about 6.4 mm to about 19.0 mm). In addition, the material from which the tampon 12 is constructed, the smoothness of the internal surface of the first member 20, the shape of the second member 22, etc. all contribute to establish a needed expulsion force to open and expel the tampon 12. This force should range from between about 250 grams to about 1,500 grams, preferably less than about 1,200 grams, and most preferably, less than about 1,000 grams. A lower force value is preferred for it assures that the tampon applicator 10 will be less susceptible to being bent or deformed as the tampon 12 is expelled. A bent applicator could cause the tampon to be inserted incorrectly. A lower force value also makes the tampon applicator 10 easier to use.

Referring again to Figs. 1 and 2, the first member 20 can have a fingergrip ring 62 located approximate the second end 30. The fingergrip ring 62 can be integrally formed from the material from which the first member 20 is constructed or it can be a separate member which is secured in place by an adhesive or some other type of attachment mechanism. The fingergrip ring 62 functions to provide a means for the user to grip the first member 20 and hold it between her thumb and middle finger. The user can then position her forefinger on the free end of the second member 22 and orient the first member 20 relative to her vagina while she pushes the second member 22 into the first member 20.

As stated above, the tampon applicator 10 includes a second member 22, also commonly referred to as an inner tube. The second member 22, like the first member 20, can be a spirally wound, a convolutely wound or a longitudinally seamed hollow tube constructed from paper, paperboard, cardboard, or a combination thereof. The second member 22 can also be formed into a cylindrical tube by overlapping the material upon itself. The second member 22 can be constructed of the same material as the first member 20 or it can be made out of a different material. Furthermore, the second member 22 could be constructed as a laminate having two or more plies which are then spirally wound, convolutely wound or longitudinally seamed into a cylindrical tube. Either a wound tube or a longitudinally seamed tube is preferred because the finished tube will have a wall 64 with a constant thickness. However, some manufacturers may prefer to construct the second member 22 as a solid stick or use some other unique shape. It is also possible to form a fingergrip ring or flange 66 on the outer end of the second member 22 to provide an enlarged surface onto which the user's forefinger can rest. The fingergrip ring 66 thereby functions as a seat for the forefinger and facilitates movement of the second member 22 into the first member 20.

Referring to Fig. 12, the second member 22 functions by being telescopically movable relative to the first member 20. As the second member 22 is pushed into the first member 20, the tampon 12 is forced forward against the pleats 50. The contact by the tampon 12 causes the pleats 50 to radially open to a diameter which is sufficient to allow the tampon 12 to be expelled from the first member 20. The open arrangement of the pleats 50 is shown in Fig. 12 after the tampon 12 has been expelled. With the tampon 12 properly positioned in the woman's vaginal cavity, the tampon applicator 10 is withdrawn and properly discarded.

The tampon applicator 10 having the semi-spherically shaped insertion tip 32 works well in combination with a catamenial tampon having a shaped nose. This is especially true when the shaped nose on the tampon 12 is configured to conform to the interior surface 48 of the insertion tip 32.

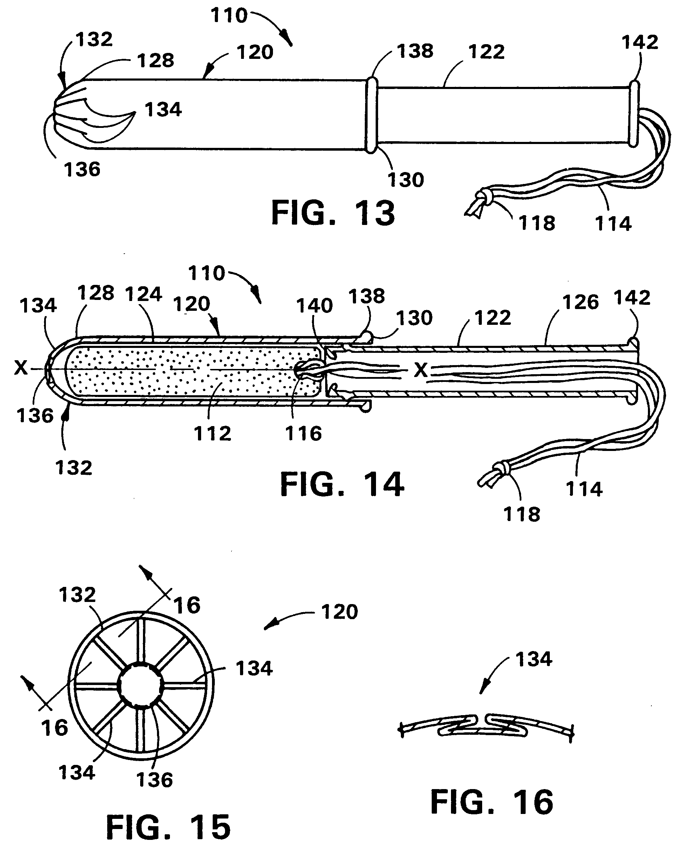

Referring to Figs. 13-15, a tampon applicator 110 is shown which is designed to house an absorbent tampon 112 and provide a comfortable means of inserting the tampon 112 into a woman's vagina. A tampon is an absorbent member primarily designed to be worn by a woman during her menstrual period to absorb menses, blood and other body fluid. The tampon 112 can be made from natural or synthetic fibers including cellulose fibers such as cotton or rayon, or artificial fibers such as polyester, polypropylene, nylon or blends thereof. Other types of fibers may also be used, such as cellulose sponge or a sponge formed from elastomeric materials. A blend of cotton and rayon fibers works well.

The tampon 112 is normally compressed into the form of a cylinder and can have a blunt, rounded or shaped forward end. The tampon 112 commonly has a withdrawal string 114 fastened to an end thereof which serves as a means for withdrawing the soiled tampon from the woman's vagina. The withdrawal string 114 is permanently affixed to the tampon 112, for example, by looping it through an aperture 116 formed transversely through the tampon 112. In addition, the withdrawal string 114 can have a knot 118 formed at it's free end to assure that the string 114 will not separate from the tampon 112.

The tampon applicator 110 includes an outer tube 120 and an inner tube 122. The outer tube 120 is preferably in the form of a spirally wound, convolutely wound or longitudinally seamed, hollow tube which is formed from paper, paperboard, cardboard or a combination thereof. The inner tube 122 can be formed from the same material as the outer tube 120, or alternatively, be made of a different material. The inner tube 122 should have a constant external diameter so as to easily slide within the inner diameter of the outer tube 120. It is also possible to construct the inner tube 122 as a solid stick, or use some other unique shape, which attaches directly to the tampon 112.

Both the outer tube 120 and the inner tube 122 are fairly rigid and commonly have a diameter of about 10 mm to about 20 mm, with the inner tube 122 being slightly smaller in diameter than the outer tube 120. The outer tube 120 has a wall 124 with a predetermined thickness of about .2 mm to about .6 mm. The inner tube has a wall 126 which is slightly thinner. The walls 124 and 126 can be constructed from a single ply of material or be formed from two or more plies which are bonded together to form a laminate. The use of two or more plies or layers is preferred for it enables the manufacturer to use certain material in the various layers which can enhance the performance of the tampon applicator 110. When two or more layers are utilized, all the layers can be spirally wound, convolutely wound or longitudinally seamed to form an elongated cylinder. The exterior surface of the wall 124 can be constructed using a smooth thin layer of material to facilitate insertion of the first member 120 into a woman's vagina.

The layers forming the walls 124 and 126 can be held together by an adhesive, such as glue, or by heat, pressure, ultrasonics, etc. The adhesive can be either water-soluble or water-insoluble. A water-soluble adhesive is preferred for environmental reasons in that the tubes 120 and 122 will quickly break apart when immersed in water. Such immersion will occur should the tubes 120 and 122 be disposed of by flushing them down a toilet. Exposure of the tubes 120 and 122 to a municipal's waste treatment plant wherein soaking in water, interaction with chemicals and agitation all occur, will cause the tubes 120 and 122 to break apart in a relatively short period of time.

The outer tube 120 is sized and configured to house the absorbent tampon 112 and the inner tube 122 is sized and configured to push the tampon 112 out of the outer tube 120. The outer tube 120 can be a straight, elongated cylindrical tube formed on a central longitudinal axis X--X. It is also possible to form the outer tube 120 into an arcuate shape. The arcuate or curved shape can assist in providing comfort when inserting the outer tube 120 into a woman's vagina. The inner tube 122 should be configured to telescopically slide in the outer tube 120. With a curved tampon applicator, it is possible to employ a curved tampon which again may be more comfortable for some women to use since the shape of the tampon may better fit the curvature of a woman's vagina.

The outer tube 120 has first and second spaced apart ends 128 and 130, respectively. The outer tube 120 can also have either a constant outer diameter or a stepped outer profile. Preferably, the outer tube 120 will have an essentially constant diameter over a major portion of it's length. Integrally formed on the first end 128 of the outer tube 120 and extending outwardly therefrom is an insertion tip 132. The insertion tip 132 is designed to facilitate insertion of the outer tube 120 into a woman's vagina in a comfortable manner. The insertion tip 132 contains a number of pleats 134 and has a semi-spherical configuration with a diameter which is approximately equal to the outside diameter of the outer tube 120. The pleats 134 can be uniformly spaced apart or they can be randomly arranged. The insertion tip 132 can have the same thickness as the outer tube 120 or be made thinner or thicker, if desired.

An aperture 136 is formed in the center of the semi-spherical shaped insertion tip 132 and is coaxially aligned with the longitudinal axis X--X. The aperture 136 can have a diameter of at least about 1.5 mm, preferably between about 1.5 to about 5.0 mm, and more preferably, between about 3.0 to about 3.5 mm. Another way of sizing the diameter of the aperture 136 is to make it less than about 30% of the diameter of the outer tube 120, preferably, between about 10% to about 30%, and most preferably, less than about 20% of the diameter of the outer tube 120. It should be noted that although the aperture 136 is described as a circle, it is possible to form the aperture 136 in other shapes such as a polygon, a square, a pentagon, a hexagon, an octagon, etc. The aperture 136 should extend entirely through the insertion tip 132. The purpose of the aperture 136 in the end of the insertion tip 132 is to facilitate the subsequent unfolding of the pleats 134 during use. The aperture 136 also assures that the pleats 134 will symmetrically open about the longitudinal axis X--X of the outer tube 120. A further benefit of the aperture 136 is that it provides a visual means for the user to inspect the tampon applicator 110 and assure herself that a tampon 112 is present in the outer tube 120.

With the aperture 136 being small, less of the absorbent tampon 112 is exposed to the vaginal tissue when the tampon applicator 110 is inserted into the woman's vagina. Since a tampon 112 is normally dry and consists of a plurality of absorbent fibers, it can cause abrasion against the walls of a woman's vagina as it is being inserted. By reducing the amount of surface area of the tampon 112 which is exposed to the vaginal tissue, one can decrease the discomfort during the insertion process. In addition, since a majority of the insertion tip 132 is closed, the frictional force between the exterior surface 126 of the outer tube 120 and the walls of the vagina is reduced. Furthermore, the small diameter of the aperture 136 also decreases the possibility of trapping or pinching vaginal tissue therein.

Referring to Figs. 15 and 16, the insertion tip 132 is shown having a plurality of pleats 134 which can radially open such that the insertion tip 132 has a diameter which is approximately equal to or larger than the diameter of the outer tube 120. The term "pleat" as used herein refers to material which is folded upon itself, for example, by doubling the material upon itself and then pressing it into place. A representative view of a pleat 134 is depicted in Fig. 16. Either an even or an odd number of pleats 34 can be present and the pleats 34 can be equally spaced apart or they can be uniformly or randomly arranged. For ease of manufacturing, it is preferred that the pleats 34 be equally spaced relative to one another. Each pleat 34 is formed by doubling the material upon itself and then pressing or adhering the material into place. Although eight equally spaced apart pleats 34 are shown in Fig. 15, it is possible to utilize various numbers of pleats 34. The number of pleats 34 can vary from between three to about thirty-two pleats, preferably between five to sixteen pleats, and most preferably, eight pleats.

Referring again to Figs. 13 and 14, the outer tube 120 can have a fingergrip ring 138 located approximate the second end 130. The fingergrip ring 138 can be integrally formed from the material from which the outer tube 120 is constructed or it can be a separate member which is secured in place by an adhesive or some other type of attachment mechanism. The fingergrip ring 138 functions to provide a means for the user to grip the outer tube 120 and hold it between her thumb and middle finger. The user can then position her forefinger on the free end of the inner tube 122 and orient the outer tube 120 relative to her vagina while she pushes the inner tube 122 into the outer tube 120.

The inner tube 122 can have an inwardly directed flange 140 formed at its forward end which provides an enlarged surface for contacting the rear end of the tampon 112. The inner tube 122 can also have a radial, outwardly extending ring 142 formed adjacent to the outer or free end of the inner tube 122 which provides an enlarged surface onto which the user's forefinger can rest. The ring 142 thereby functions as a seat for the forefinger and facilitates movement of the inner tube 122 into the outer tube 120.

The inner tube 122 functions by being telescopically movable relative to the outer tube 120. As the inner tube 122 is pushed into the outer tube 120, the tampon 112 is forced forward against the pleats 134. The contact by the tampon 112 causes the pleats 134 to radially open to a diameter which is sufficient to allow the tampon 112 to be expelled from the outer tube 120. With the tampon 112 properly positioned in the woman's vagina, the tampon applicator 110 is withdrawn and properly discarded.

### APPARATUS

The outer tube 120 can have the insertion tip 132 formed into the desired semi-spherical configuration with the central aperture 136 by using the apparatus described below.

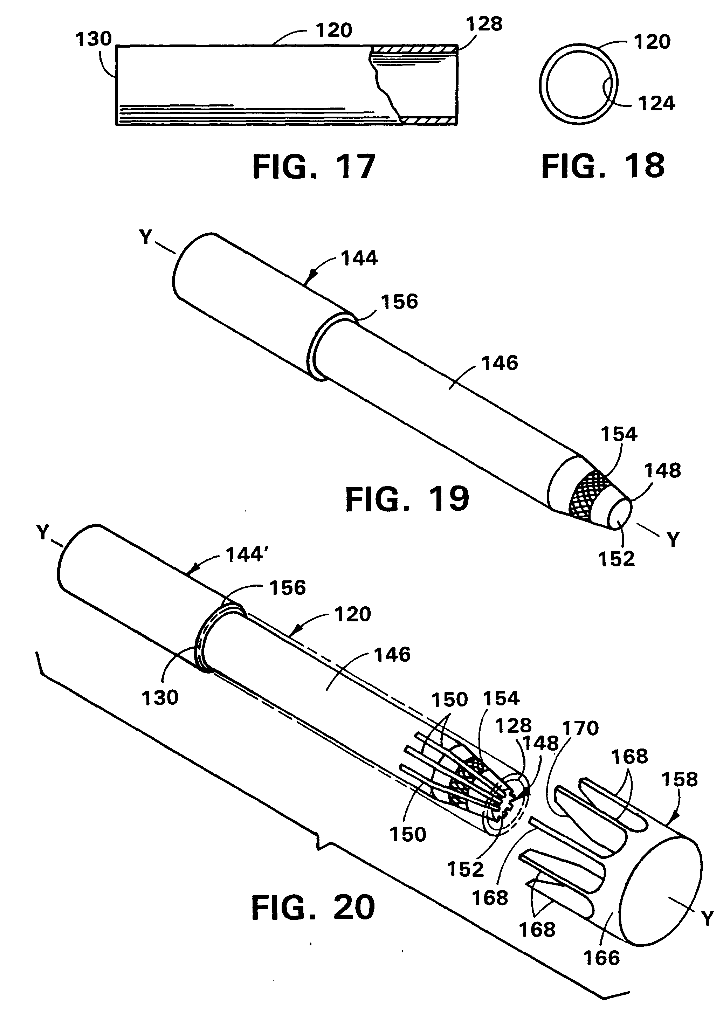

Referring to Figs. 17 and 18, the outer tube 120 is shown before the insertion tip 132 is formed. At this stage, the outer tube 120 has an essentially constant inside diameter and the wall 124 has a constant thickness.

Referring to Figs. 19 and 20, a first punch 144 is shown having a tubular section 146 which is sized and configured to receive the outer tube 120. In other words, the outer tube 120 must be able to slide onto the tubular section 146 with only a small amount of clearance therebetween. The first punch 144 has a tip 148. The tip 148 can be smooth or void of any grooves as shown in Fig. 19. Alternatively, the first punch 144' can have a configured tip 148 with a plurality of elongated grooves 150 formed therein, as is depicted in Fig. 20. When the grooves 150 are present, there should be at least four grooves 150, preferably between eight to twelve grooves 150, with eight grooves 150 being most preferred. The purpose of the grooves 150 will be explained shortly.

The tip 148 can be formed into a frusto-conical shape having a blunt end 152. Other shapes can also be utilized if desired. At least a portion of the exterior surface of the tip 148 can be knurled 154 to provide a frictional surface between the tip 148 and the inner surface of the outer tube 120 as the insertion tip 132 is being formed. A medium knurl 154 will provide an adequate frictional surface for the crimping operation. The first punch 144 or 144' also has a shoulder 156 formed at an opposite end of the tubular section 146 which acts as a stop for the outer tube 120. It should be noted that the length of the tubular section 146 is sized to conform closely to the length of the outer tube 120. A typical outer tube 120 will have a length of between about 2 inches to about 4 inches (about 50.8 mm to about 101.6 mm), preferably about 3 inches to about 3.5 inches (about 76.2 mm to about 88.9 mm), most preferably, at least about 3.12 inches (about 79.2 mm). The tubular section 146 should have a length which is equal to or slightly greater than the initial length of the outer tube 120 as shown in Fig. 17. The first end 128 of the tube 120 can be aligned approximately flush with the tip 148 when the tube 120 is positioned on the tubular section 146. However, an extra length of about 0.06 inches (about 1.5 mm) on the tubular section 146 of the first punch 144 or 144' is advantageous for permitting the first punch 144 or 144' to mate with a first die 158.

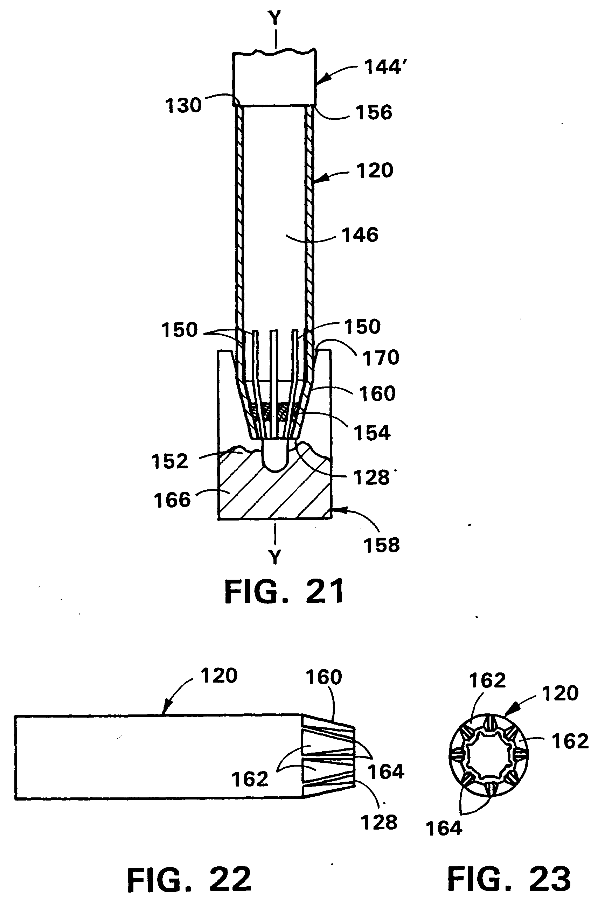

Referring to Figs. 20 and 21, the first punch 144' is shown with the outer tube 120 slid onto the tubular section 146 such that the second end 130 of the outer tube 120 abuts the shoulder 156 and is mateable with the first die 158. The axial engagement of both the first punch 144' and the outer tube 120 with the first die 158 enables the tip 132 of the outer tube 120 to be crimped. The term "crimped" as used herein refers to pressing or pinching the material forming the insertion tip 132 into small, regular folds or ridges with troughs therebetween.

Referring to Figs. 22 and 23, the crimped tip 160 consists of a plurality of ridges 162 and troughs 164 formed about the circumference of the first end 128 of the outer tube 120. The troughs 164 are the deepest adjacent the first end 128 and become shallower as the troughs move away from the first end 128. The ridges 162 are formed on a circle having a smaller diameter adjacent the first end 128 and expand outward as the ridges 162 move away from the first end 128.

Referring again to Figs. 20 and 21, the crimped tip 160 is formed by the engagement of the first punch 144 or 144' with the first die 158. The first die 158 includes a base 166 having a plurality of blades 168 extending axially outward therefrom. There should be at least four blades 168, preferably, between eight to twelve blades, with eight blades being the most preferred. For best results with hard board papers, each groove 150 formed in the first punch 144' should align with à blade 168 formed in the first die 158. Either an even number or an odd number of blades 168 can be utilized. An even number of blades 168 are easier to machine and the first die 158 will then have a symmetrical shape, which is also advantageous. For example, symmetrically shaped dies can be measured across their tips to determine their size.

The blades 168 can range from about .5 inches (about 12.7 mm) to about 2 inches (about 50.8 mm) in length. A length of approximately 1 inch (25.4 mm) is sufficient. The blades 168 have an angled or tapered inner surface 170 which enables then to mate with the smooth tip 148 formed on the first punch 144 or mate with and axially enter a corresponding groove 150 formed in the first punch 144'. The angle can vary depending upon the taper on the smooth tip 148 or depending on the angle at which each corresponding groove 150 is formed. The angle on each blade 168 can be equal to or different from the angle formed on the smooth tip 148 and can also be equal to or different from the angle to which each groove 150 is machined. When the grooves 150 are present, each groove 150 should be sized to be larger than the corresponding blade 168 so that the thickness of wall 124 of the outer tube 120 can also be received into the grooves 150. Each blade 168 is machined to an angle which is different from the angle to which the bottom of the grooves 150 have been machined to. When the first punch 144' is fully inserted into the first die 158, the angled surfaces 170 of each blade 168 is spaced apart from the bottom of each corresponding groove 150. This clearance permits the wall thickness of the outer tube 120 to be sandwiched therebetween and provides the undulating surface which is the crimp 160 shown in Fig. 23. The angle that each groove 150 and each blade 168 is formed at can vary. The grooves 150 and the blades 168 can be formed at identical angles relative to a longitudinal central axis Y--Y of the first punch 144' and the first die 158, or they can be formed at different angles relative to one another. For outer tubes 120 formed from hard paperboard, good quality pleats 134 can be formed using the first punch 144 with the smooth tip 148 mating with the first die 158. For softer paperboard, it is advantageous to machine an angle of about 20° to the Y--Y axis in the first die 158 and to machine an angle of about 15° to the Y--Y axis in the first punch 144'.

Referring to Fig. 21, one can see the position of the outer tube 120 on the first punch 144' while the first end 128 is crimped. The knurled surface 154 serves to prevent the outer tube 120 from moving toward the shoulder 156 when the first punch 144' engages the first die 158. It has been found that the force exerted on the outer tube 120 increases as the first punch 144 or 144' engages deeper and deeper into the first die 158. When the knurled surface 154 is not present, this force can drive the outer tube 120 up against the shoulder 156 and cause the second end 130 of the outer tube 120 to become wrinkled and radially enlarged. Such a feature is not aesthetically pleasing and must be avoided.

After the outer tube 120 has a crimped tip 160 formed on the first end 128 thereof, the first punch 144 or 144' and the first die 158 are separated and the outer tube 120 is removed.

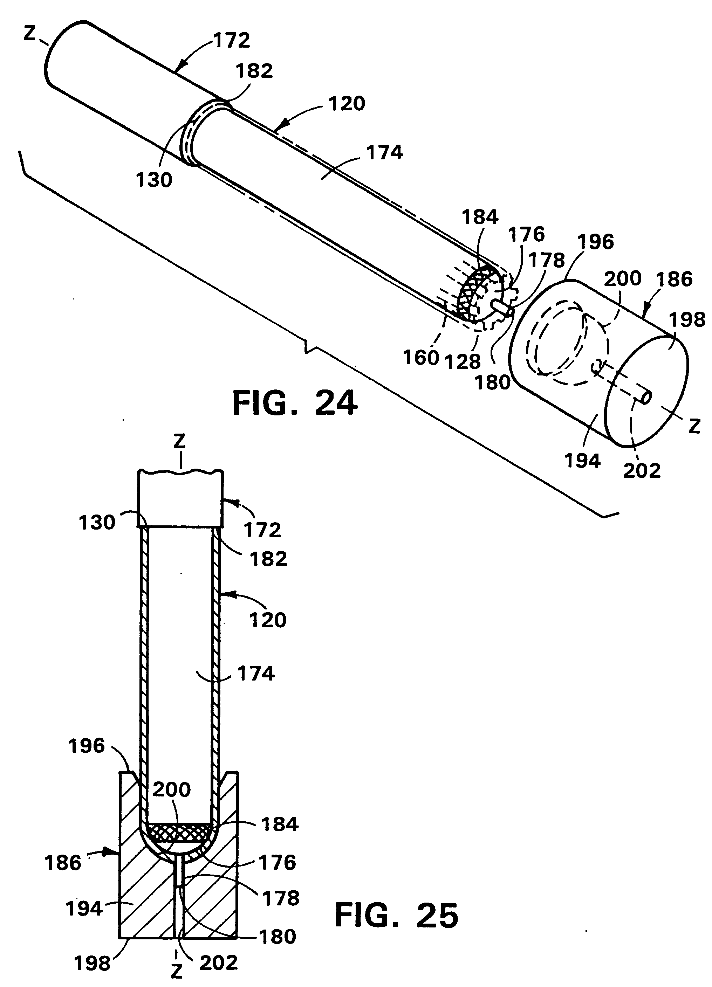

Referring to Figs. 24 and 25, the outer tube 120 is then subjected to a second operation wherein the crimped tip 160 is pleated and pressed into a semi-spherical configuration. This is accomplished by using a second punch 172 having a tubular section 174 which is sized to receive the outer tube 120. The second punch 172 has a semi-spherically shaped tip 176 with a pin 178 extending outward from the apex of the tip 176. The pin 178 has a distal or free end 180. The second punch 172 also contains a shoulder 182 formed at an opposite end of the tubular section 174 which acts as a stop for the outer tube 120. It should be noted that the length of the tubular section 174 is sized to conform closely to the length of the outer tube 120. The second punch 172 is designed to have the outer tube 120 slid over the tubular section 174 until the second end 130 of the outer tube 120 abuts against the shoulder 182. In this position, the crimped tip 160 formed on the first end 128 of the outer tube 120 should extend about 0.06 inches to about 0.12 inches (about 1.5 mm to about 3.0 mm) beyond the apex of the semi-spherically shaped tip 176. The free end 180 of the pin 178, however, will extend beyond the crimped end 160 by at least 0.06 inches (1.5 mm), and preferably more.

At least a portion of the semi-spherically shaped tip 176 can be knurled 184 to provide a frictional surface between the tip 176 and the inner surface of the outer tube 120 as the insertion tip 132 is being formed. A medium knurl 184 will provide an adequate frictional surface for the pleating and forming operation. The knurled surface 184 serves to prevent the outer tube 120 from moving toward the shoulder 182 of the second punch 172.

The pin 178 can have a length of at least 0.06 inches (1.5 mm) but is preferably longer. The pin 178 should have a diameter of at least 0.062 inches (1.5 mm), preferably at least 0.125 inches (3.1 mm), and can be larger if desired. The cross-section of the pin 178 is preferably circular but could be of a different configuration if desired. A circular cross-section is preferred for it forms an opening with a circular periphery. A circular opening is aesthetically pleasing to the eye and since one of the purposes of the aperture 136 is to allow the consumer to see if a tampon 112 is present in the tampon applicator 110, the aperture could be circular. The pin 178 is shown having an essentially constant outside diameter. However, it is possible to form the pin 178 such that it tapers down in diameter from a larger diameter located adjacent to the point of attachment to the apex of the semi-spherical tip 176 to a smaller diameter adjacent the free end 180.

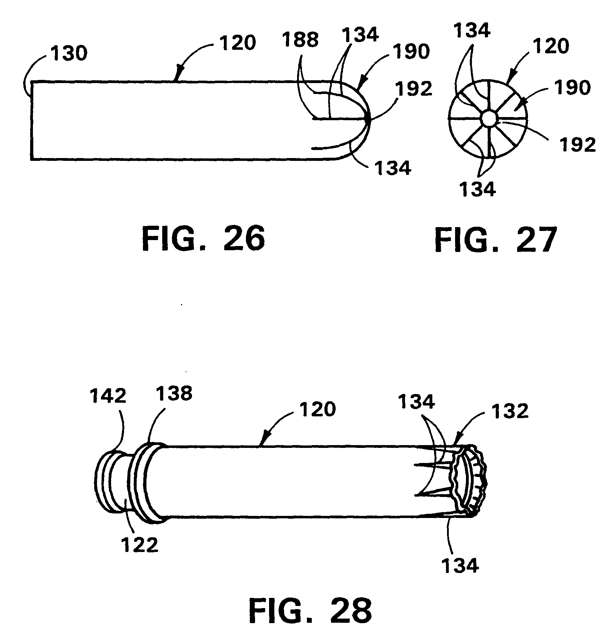

The second punch 172 is sized and configured to engage with a second die 186 so as to transform the crimped tip 160 of the outer tube 120 into a plurality of pleats 134 and form the pleats 134 into a semi-spherically shaped tip 190, see Figs. 26 and 27. The pleats 134 can be uniformly or randomly spaced apart and can have a dovetail-like appearance. The length of each pleat 134 should be aligned approximately straight with or parallel to the longitudinal axis of the tube 120 versus being undulating or curved. A straight pleat normally requires a lesser amount of force to open. The pleats 134 should terminate at a point 188 which is approximately tangent to the point where the semi-spherically shaped tip 190 joins to the exterior surface of the outer tube 120. The semi-spherically shaped tip 190 will have a central aperture 192 formed therethrough because of the presence of the pin 178. The aperture,192 allows the consumer to visually inspect the tampon applicator 10 to see if a tampon 112 is present. The aperture 192 can vary with the diameter of the tube 120 but should not be so large that it would allow a woman to feel it as she inserts the tampon 112 into her vagina. If the aperture 192 is too large, it could cause discomfort as the woman inserts the tampon applicator 110 into her vagina.

Referring again to Figs. 24 and 25, the second die 186 includes a base 194 having a first end 196 and a second end 198. A semi-spherically shaped cavity 200 formed in the base 194 adjacent to the first end 196. The semi-spherically shaped cavity 200 is sized to receive the semi-spherically shaped tip 176 formed on the outer tube 120 as well as the wall thickness of the outer tube 120. This difference in size will allow the insertion tip 132 to be formed on the first end 128 of the outer tube 120. The surface of the cavity 200 is preferably polished to improve the appearance of the finished semi-spherically shaped tip 176 and to facilitate removal of the finished tube 120 from the second die 186. The polished surface can have a "surface roughness average" value of between about 0.1 µm to about 0.4 µm (about 4 micro inches to about 16 micro inches).

The base 194 also has a central passageway 202 formed therein which is axially aligned along a longitudinal central axis Z--Z. The passageway 202 extends from the bottom of the cavity 200 to the second end 198. If desired, the passageway 202 can be a closed passageway which terminates short of the second end 198. The passageway 202 is sized and configured to receive only the pin 178. The outside diameter of the pin 178 should be slightly smaller than the inside diameter of the passageway 202. The relationship between the mating second punch 172, the outer tube 120 and the second die 186 is clearly shown in Fig. 25.

It should be noted that both the length and diameter of commercially available tampons do vary and therefore the tampon applicator 110 should be manufactured in a variety of sizes. Tampons can vary in length from about 1 inch to about 3 inches (about 25.4 mm to about 76.2 mm) but preferably are about 2 inches (about 50.8 mm) in length. The tampon diameter will also vary from about .25 inches to about .75 inches (about 6.4 mm to about 19.0 mm). In addition, the material from which the tampon 112 is constructed, the smoothness of the internal surface of the outer tube 120, the shape of the inner tube 122, etc. all contribute to establish a needed expulsion force to open and expel the tampon 112. This force should range from between about 250 grams to about 1,500 grams, preferably less than about 1,200 grams, and most preferably, less than about 1,000 grams. A lower force value is preferred for it assures that the tampon applicator 110 will be less susceptible to being bent or deformed as the tampon 112 is expelled. A bent applicator could cause the tampon to be inserted incorrectly. A lower force value also makes the tampon applicator 110 easier to use. The size of the aperture 192 will also affect the amount of force needed to open the pleats 134. Typically, the larger the diameter of the aperture 192, the lower the force required to open the pleats 134.

### METHOD

The method of crimping, pleating and forming a semi-spherically shaped tip 190 on a hollow tube 120 is as follows, using the above-identified punches 144, 144' and 172, and dies 158 and 186. The above identified punches 144, 144' and 172, and dies 158 and 186 can be manually or automatically engaged and disengaged to form the insertion tip 132 on the outer tube 120. It is contemplated that the punches 144, 144' and 172, and the dies 158 and 186 will be actuated at sufficient speeds to crimp, pleat and form in excess of 100, preferably in excess of 300, and most preferably, more than 500 outer tubes per minute.

The method involves sliding the hollow tube 120 onto the first punch 144 or 144' until one end 130 of the tube 120 contacts the shoulder 156. The first punch 144 or 144' and the tube 120 are moved into engagement with the first die 158 and a plurality of crimps are formed on the opposite end 128 of the tube 120. The plurality of crimps form the crimped end 160. The first punch 144 or 144' is then disengaged from the first die 158 and the tube 120 having the crimped end 160 is removed from the first punch 144 or 144'. The tube 120 is then slid onto the second punch 172 until the non-crimped end 130 of the tube 120 contacts the shoulder 182. The second punch 172 and the tube 120 are brought into engagement with the second die 186 thereby allowing the axially extending pin 178 to enter the passageway 202. The mating of the second punch 172 with the second die 186 transforms the crimped end 160 of the tube 120 into a plurality of pleats 134 and forms the pleats 134 into a semi-spherically shaped tip 190 having a central aperture 192 formed therethrough. Once the tip 190 is formed, the second punch 172 is disengaged from the second die 186 and the outer tube 120 is removed from the second punch 172.

## Claims

1. A tampon applicator (10) comprising:
a) a first member (20) capable of housing an absorbent tampon (12), said first member (20) having a central longitudinal axis and first (28) and second ends (30);
b) an insertion tip (32) integrally formed on said first end (28) of said first member (20) and extending outwardly therefrom, said insertion tip (32) including a plurality of pleats (50), said pleats (50) capable of expanding outward as said tampon (12) is expelled from said first member (20); and
c) a second member (22) telescopically mounted in said second end (30) of said first member (20), said second member (22) adapted to expel said tampon (12) through said insertion tip (32) as it is pushed into said first member (20) ,
said tampon applicator (10) characterised in that said insertion tip (32) has an aperture (36) extending therethrough with a side wall (38), said side wall (38) being aligned parallel to said central longitudinal axis, said aperture having a diameter between 1.5 mm to 5.0 mm and said plurality of pleats (50) is arranged to form a semi-spherical configuration,
wherein the cross-section of the semi-spherical configuration spans an arc (A) of approximately 180 degrees and wherein the semi-spherical configuration is formed on an outside diameter which is sized to be equal to or slightly smaller than the outside diameter of the first member (20).

2. The tampon applicator (10) of claim 1, wherein said first member (20) is a spirally wound, hollow paper tube.

3. The tampon applicator (10) of claim 1, wherein said first member (20) is a convolutely wound, hollow paper tube.

4. The tampon applicator (10) of claim 2, wherein said first member (20) is a longitudinally seamed, hollow paper tube.

5. The tampon applicator (10) of claim 4, wherein said paper tube is constructed from at least one ply of material.

6. The tampon applicator (10) of claim 1, wherein said insertion tip (32) contains an even number of pleats (50).

7. The tampon applicator (10) of claim 1, wherein said insertion tip (32) contains an odd number of pleats (50).

8. The tampon applicator (10) of claim 1, wherein said insertion tip (32) contains from between three to thirty-two pleats (50).

9. The tampon applicator (10) of claim 1, wherein said insertion tip (32) contains from between 6 to 12 pleats (50).

10. A tampon applicator (10) according to one of the preceding claims, wherein said first member (20) has a substantially smooth exterior surface and said insertion tip (32) includes at least three pleats (50) arranged to form a semi-spherical configuration, said pleats (50) capable of expanding radially outward as said tampon (12) is expelled from said first member (20).

11. The tampon applicator (10) of claim 10, wherein each of said pleats (50) has a first end (52) which coincides with said aperture (36) and a second end (54) which coincides with a point on an exterior surface (26) of said insertion tip (32).

12. The tampon applicator (10) of claim 10, wherein each of said pleats (50) has a first end (52) which coincides with said aperture (36) and a second end (54) which coincides at a location where said insertion tip (32) is integrally joined to said first member (20).

13. The tampon applicator (10) of claim 10, wherein each of said pleats (50) has a first end (52) which coincides with said aperture (36) and a second end (54) which coincides with a point on said exterior surface (26) of said first member (20).

14. The tampon applicator (10) of claim 10, wherein said insertion tip (32) contains 8 pleats (50) each spaced approximately an equal distance apart.

15. The tampon applicator (10) according to claims 1 to 6 and 8 to 14, wherein said first member (20) is a hollow spirally wound tube, said first member having a substantially smooth exterior surface and said insertion tip (32) includes eight pleats (50) spaced approximately an equal distance apart.

16. The tampon applicator of claim 15, wherein said aperture (36) has a diameter of about 3.0 mm.

17. The tampon applicator of claim 15, wherein said side wall of said aperture (36) contains a radius adjacent to an exterior surface of said insertion tip (32).

18. The tampon applicator (10) of claim 15, wherein said pleats (50) have a thickness of less than 0.5 mm.

19. In combination, a tampon applicator (110) and a catamenial tampon (112) having a shaped nose, said combination comprising:
a) a first member (120) capable of housing said catamenial tampon (112), said first member (120) having a central longitudinal axis and first (128) and second ends (130) ;
b) an insertion tip (132) integrally formed on said first end (128) of said first member (120) and extending outwardly therefrom, said insertion tip (132) including a plurality of pleats (134), said pleats (134) capable of expanding outward as said tampon (112) is expelled from said first member (120); and
c) a second member (122) telescopically mounted in said second end (130) of said first member (120), said second member (122) adapted to expel said tampon (112) through said insertion tip (132) as it is pushed into said first member (120) ;
said combination characterised in that said insertion tip (132) of said tampon applicator (110) has an aperture (136) extending therethrough with a side wall said side wall (38) being aligned parallel to said central longitudinal axis, said aperture having a diameter between 1.5 mm to 5.0 mm, and wherein said plurality of pleats (134) is arranged to form a semi-spherical configuration.

20. The combination of claim 19 wherein said insertion tip (132) has a semi-spherically shaped interior surface and said shaped nose on said tampon is configured to conform to said interior surface.

21. An apparatus for crimping, pleating and forming a tip (132) on a hollow tube (120), said apparatus comprising:
a) a first punch (144) having a tubular section (146) sized to be introduced into said tube (120) and shoulder (156) formed at an opposite end of said tubular section (146) which acts as a stop for said tube (120);
b) a first die (158) mateable with said first punch (144) and tube (120) to crimp an end of said tube (120), said first die (158) including a base (166) having a plurality of blades (168) extending axially outward therefrom, each of said blades (168) capable of engaging said tube (120) and crimping the tip (132) of said tube (120) positioned therebetween;
c) a second punch (172) having a tubular section (174) sized to be introduced into said tube (120) and having a semi-spherically shaped tip (176) with a pin (178) extending outward from the apex of said tip (176), said pin having a diameter of at least 1.5 mm, said second punch (172) further having a shoulder (182) formed at an opposite end of said tubular section (174) which acts as a stop for said tube (120); and
d) a second die (186) mateable with said second punch (172) to transform said crimped end of said tube (120) into a plurality of pleats (134) and form said pleats (134) into a semi-spherically shaped tip (190) having a central aperture (192) formed therethrough, said second die (186) including a base (194) having a semi-spherical cavity (200) formed therein with a central passageway (202) formed at the bottom of said cavity (200), said cavity (200) being sized to receive both said second punch (172) and said tube (120) and said passageway (202) being sized to receive only said pin (178).

22. The apparatus of claim 21, wherein said pin (178) has a diameter of at least 0.32 cm (0.125 inches).

23. The apparatus of claim 21, wherein said pin (178) has a circular cross-sectional configuration.

24. The apparatus according to claims 21 to 23, wherein said first punch (144) further has a configured tip (148) with a plurality of elongated grooves (150) formed therein, and wherein said first die (158) comprises a plurality of blades (168) wherein each of said blades (168) is capable of engaging with one of said groove (150) formed on said first punch (144).

25. The apparatus of claim 24, wherein said configured tip (148) formed on said first punch (144) contains a knurled area (154) which facilitates crimping said tube (120) when said first punch (144) is brought in contact with said first die (158).

26. The apparatus of claim 24, wherein said semi-spherically shaped tip (176) formed on said second punch (172) contains a knurled area (184) which facilitates pleating and forming said tube (120) when said second punch (172) is brought in contact with said second die (186).

27. The apparatus of claim 24, wherein said first die (158) contains at least four blades (168).

28. The apparatus of claim 24, wherein said first die (158) contains at least 8 blades (168).

29. The apparatus of claim 24, wherein said first punch (144) contains at least four elongated grooves (150).

30. The apparatus according to claims 24 to 29, wherein said first punch (144) has a configured tip (148) with eight elongated grooves (150) formed therein; wherein said first die (158) includes a base (166) having eight blades (168) extending axially outward therefrom, and wherein said second die (186) is mateable with said second punch (172) to transform said crimped end of said tube (120) into eight pleats (134) and form said eight pleats (134) into a semi-spherically shaped tip (190) having a central aperture (192) formed therethrough.

31. The apparatus of claim 30, wherein said pin (178) has a diameter of at least 0.16 cm (0.062 inches).

32. The apparatus of claim 30, wherein said pin (178) has an essentially constant diameter.

33. The apparatus of claim 30, wherein said pin (178) has a free end and said pin tapers in diameter down from a larger diameter located adjacent to its point of attachment to said tip to a smaller diameter at its free end.

34. The apparatus of claim 30, wherein said pin (178) has a length of at least 0.15 cm (0.06 inches).

35. A method of crimping, pleating and forming a tip (190) on a hollow tube (120) using a first punch (144, 144') having a tubular section sized to be introduced into said tube (120) and having a configured tip with a plurality of elongated grooves formed therein, and a shoulder (156) formed at an opposite end of said tubular section which acts as a stop for said tube (120), a first die (158) mateable with said first punch (144, 144') and tube (120) to crimp an end of said tube (120) said first die (158) including a base having a plurality of blades extending axially outward therefrom, each of said blades capable of engaging with one of said grooves formed on said first punch (144, 144') and crimping the tip of said tube (120) positioned therebetween, a second punch (172) having a tubular section sized to be introduced into said tube and having a semi-spherically shaped tip with a pin (178) extending outward from the apex of said tip, said pin having a diameter of at least 1.5 mm, said second punch (172) further having a shoulder (182) formed at an opposite end of said tubular section which acts as a stop for said tube (120) and a second die (186) mateable with said second punch (172) to transform said crimped end of said tube (120) into a plurality of pleats (134) and form said pleats (134) into a semi-spherically shaped tip (190) having a central aperture (192) formed therethrough, said aperture having a diameter between 1.5 mm to 5.0 mm, said second die (186) including a base having a semi-spherical cavity formed therein with a central passageway formed at the bottom of said cavity, said cavity being sized to receive only said pin (178), said method comprising the steps of:
a) sliding said hollow tube (120) onto said first punch (144, 144') until an end of said tube (120) contacts said shoulder (156);
b) moving said first punch (144, 144') and tube (120) into engagement with said first die (158) to form a plurality of crimps on an opposite end of said tube (120);
c) disengaging said first punch (144, 144') from said first die (158) and removing said crimped tube from said first punch;
d) sliding said tube (120) onto said second punch (172) until the non-crimped end of said tube (120) contacts said shoulder (182);
e) moving said second punch (172) and tube (120) into engagement with said second die (186) and engaging said pin (178) in said passageway to transform said crimped end of said tube (120) into a plurality of pleats (134) and forming said pleats (134) into a semi-spherically shaped tip (190) having a central aperture (192) formed therethrough; and
f) disengaging said second punch from said second die and removing said pleated and formed tube (120) from said second punch (172).

36. The method of claim 35, wherein when one end of said tube (120) contacts said shoulder, the opposite end of said tube is approximately flush with said configured tip formed on said first punch (144, 144').

37. The method of claim 35, wherein said first punch (144, 144') and said first die (158) are engaged to form at least four pleats in one end of said tube (120).

38. The method of claim 35, wherein said second punch (172) and said second die are engaged to form an aperture at one end of said tube (120) having a circular periphery.

39. The method of claim 35, wherein said first punch (144, 144') and said first die (158) are axially engaged such that each of said blades formed on said first die are axially aligned with one of said corresponding grooves formed on said first punch.

## Patentansprüche

1. Tamponapplikator (10) umfassend:
a) ein erstes Element (20), fähig zum Unterbringen eines Absorbtionstampons (12), wobei das erste Element (20) eine zentrale Längsachse und ein erstes (28) und ein zweites Ende (30) umfasst;
b) eine Einführspitze (32), integriert gebildet auf dem sich von dort nach außen ausdehnenden ersten Endes (28) des ersten Elements (20), wobei die Einführspitze (32) mehrere Falten (50) einschließt, wobei diese Falten (50) fähig sind, sich nach außen auszudehnen, wenn der Tampon (12) aus dem ersten Element (20) hinausgeschoben wird; und
c) ein zweites Element (22), teleskopisch in dem zweiten Ende (30) des ersten Elements (20) befestigt, wobei das zweite Element (22) angepasst ist, um den Tampon (12) durch die Einführspitze (32) hinauszuschieben, wenn er in das erste Element (20) gedrückt wird,
wobei der Tamponapplikator (10) dadurch gekennzeichnet ist, dass die Einführspitze (32) eine sich dahindurch erstreckende Öffnung (36) mit einer Seitenwand (38) aufweist, wobei die Seitenwand (38) parallel zu der zentralen Längsachse ausgerichtet ist, wobei die Öffnung einen Durchmesser zwischen 1.5 mm und 5.0 mm aufweist und mehrere Falten (50) so angeordnet sind, dass sie eine halbsphärische Konfiguration bilden, wobei der Querschnitt der halbsphärischen Konfiguration einen Bogen (A) von etwa 180 Grad spannt, und
wobei die halbsphärische Konfiguration auf einem Außendurchmesser gebildet ist, welcher gleich oder etwas kleiner als der Außendurchmesser des ersten Elements (20) ist.

2. Tamponapplikator (10) gemäß Anspruch 1, wobei das erste Element (20) eine spiralförmig gewickelte, hohle Papierröhre ist.

3. Tamponapplikator (10) gemäß Anspruch 1, wobei das erste Element (20) eine ringförmig gewickelte, hohle Papierröhre ist.

4. Tamponapplikator (10) gemäß Anspruch 2, wobei das erste Element (20) eine der Länge nach genähte, hohle Papierröhre ist.

5. Tamponapplikator (10) gemäß Anspruch 4, wobei die Papierröhre aus wenigstens einer Lage Material besteht.

6. Tamponapplikator (10) gemäß Anspruch 1, wobei die Einführspitze (32) eine gerade Anzahl an Falten (50) beinhaltet.

7. Tamponapplikator (10) gemäß Anspruch 1, wobei die Einführspitze (32) eine ungerade Anzahl an Falten (50) beinhaltet.

8. Tamponapplikator (10) gemäß Anspruch 1, wobei die Einführspitze (32) zwischen drei und zweiunddreißig Falten (50) beinhaltet.

9. Tamponapplikator (10) gemäß Anspruch 1, wobei die Einführspitze (32) zwischen 6 und 12 Falten (50) beinhaltet.

10. Tamponapplikator (10) gemäß einem der vorhergehenden Ansprüche, wobei das erste Element (20) eine im Wesentlichen weiche Außenoberfläche aufweist und die Einführspitze (32) wenigstens drei Falten (50) enthält, welche so angeordnet sind, dass sie eine halbsphärische Konfiguration bilden, wobei die Falten (50) fähig sind, sich radial nach Außen auszudehnen, wenn der Tampon (12) aus dem ersten Element (20) hinausgeschoben wird.

11. Tamponapplikator (10) gemäß Anspruch 10, wobei jede der Falten (50) ein genau mit der Öffnung (36) übereinstimmendes erstes Ende (52) und ein mit einem Punkt auf einer Außenoberfläche (26) der Einführspitze (32) übereinstimmendes zweites Ende (54) aufweist.

12. Tamponapplikator (10) gemäß Anspruch 10, wobei jede der Falten (50) ein genau mit der Öffnung (36) übereinstimmendes erstes Ende (52) und ein zweites Ende (54) aufweist, welches mit einer Stelle übereinstimmt, wo die Einführspitze (32) mit dem ersten Element (20) integriert verbunden ist.

13. Tamponapplikator (10) gemäß Anspruch 10, wobei jede der Falten (50) ein genau mit der Öffnung (36) übereinstimmendes erstes Ende (52) und ein zweites Ende (54) aufweist, welches mit einem Punkt auf der Außenoberfläche (26) des ersten Elements (20) übereinstimmt.

14. Tamponapplikator (10) gemäß Anspruch 10, wobei die Einführspitze (32) 8 Falten (50) beinhaltet, die alle etwa mit gleicher Distanz voneinander beabstandet sind.

15. Tamponapplikator (10) gemäß den Ansprüchen 1 bis 6 und 8 bis 14, wobei das erste Element (20) eine hohle, spiralförmig gewickelte Röhre ist, wobei das erste Element eine im Wesentlichen weiche Außenoberfläche aufweist und die Einführspitze (32) acht alle etwa mit gleicher Distanz voneinander beabstandete Falten (50) enthält.

16. Tamponapplikator gemäß Anspruch 15, wobei die Öffnung (36) einen Durchmesser von etwa 3.0 mm aufweist.

17. Tamponapplikator gemäß Anspruch 15, wobei die Seitenwand der Öffnung (36) einen Radius benachbart zu einer Außenoberfläche der Einführspitze (32) beinhaltet.

18. Tamponapplikator (10) gemäß Anspruch 15, wobei die Falten (50) eine Dicke von weniger als 0.5 mm aufweisen.

19. Kombination aus einem Tamponapplikator (110) und einem Menstruationstampon (112) mit einer geformten Nase, wobei diese Kombination umfasst:
a) ein erstes Element (120), welches zum Unterbringen des Menstruationstampons (112) fähig ist, wobei das erste Element (120) eine zentrale Längsachse und ein erstes (128) und ein zweites Ende (130) aufweist;
b) eine Einführspitze (132), integriert gebildet auf einem sich von dort nach außen ausdehnenden ersten Endes (128) des ersten Elements (120), wobei die Einführspitze (132) mehrere Falten (134) einschließt, wobei diese Falten (134) fähig sind, sich nach außen auszudehnen, wenn der Tampon (112) aus dem ersten Element (120) hinausgeschoben wird; und
c) ein zweites Element (122) teleskopisch an dem zweiten Ende (130) des ersten Elements (120) befestigt, wobei das zweite Element (122) angepasst ist, um den Tampon (112) durch die Einführspitze (132) hinauszuschieben, wenn er in das erste Element (120) gedrückt wird;
wobei diese Kombination sich dadurch auszeichnet, dass die Einführspitze (132) des Tamponapplikators (110) eine Öffnung (136) dahindurch ausdehnend, mit einer Seitenwand aufweist,
wobei die Seitenwand (38) parallel zur zentralen Längsachse verläuft, wobei die Öffnung einen Durchmesser zwischen 1.5 mm und 5.0 mm aufweist, und wobei die Mehrzahl von Falten (134) so angeordnet ist, dass sie eine halbsphärische Konfiguration bildet.

20. Kombination gemäß Anspruch 19, wobei die Einführspitze (132) eine halbsphärische geformte Innenoberfläche aufweist und die geformte Nase auf dem Tampon so gestaltet ist, dass sie mit der Innenoberfläche übereinstimmt.

21. Vorrichtung zum Kräuseln, Falten und Bilden einer Spitze (132) auf einer Hohlröhre (120), wobei diese Vorrichtung umfasst:
a) einen ersten Stempel (144), welcher einen röhrenförmigen Abschnitt (146) aufweist, welcher eine derartige Größe hat, dass er in die Röhre (120) eingeführt werden kann, und eine Schulter (156), welche auf einem anderen Ende des röhrenförmigen Abschnitts (146) gebildet ist und als Haltepunkt dieser Röhre (120) dient.
b) eine erste Form (158), welche ein Gegenstück zu dem ersten Stempel (144) darstellt, und eine Röhre (120), um ein Ende der Röhre (120) zu kräuseln, wobei die erste Form (158) eine Basis (166) enthält, welche eine Mehrzahl von Flügeln (168) aufweist, die sich von dort aus nach außen axial ausdehnen, wobei jeder der Flügel (168) fähig ist, die Röhre (120) in Eingriff zu bringen und die Spitze (132) der Röhre (120), welche dazwischen liegt, zu kräuseln;
c) einen zweiten Stempel (172), welcher einen röhrenförmigen Abschnitt (174) aufweist, welcher eine derartige Größe hat, dass er in die Röhre (120) eingeführt werden kann, und welcher eine halbsphärisch geformte Spitze (176) mit einem Stift (178) aufweist, der sich vom Scheitelpunkt der Spitze (176) nach außen ausdehnt, wobei der Stift einen Durchmesser von wenigstens 1.5 mm aufweist, wobei der zweite Stempel (172) weiterhin eine Schulter (182) aufweist, die am gegenüberliegenden Ende des röhrenförmigen Abschnitts (174) gebildet ist, welcher als Haltepunkt für die Röhre (120) dient; und
d) eine zweite Form (186), welche ein Gegenstück zum zweiten Stempel (172) darstellt, um das gekräuselte Ende der Röhre (120) in mehrere Falten (134) zu überführen, und um die Falten (134) in eine halbsphärisch geformte Spitze (190) zu formen, welche eine zentrale Öffnung (192) aufweist, die dahindurch geformt ist, wobei die zweite Form (186) eine Basis (194) enthält, die einen halbsphärischen Hohlraum (200) aufweist, der darin einen am Boden der Öffnung (200) gebildeten zentralen Durchgang aufweist, wobei der Hohlraum (200) eine derartige Größe hat, um sowohl den zweiten Stempel (172) als auch die Röhre (120) aufzunehmen, und wobei der Durchgang (202) eine derartige Größe hat, um nur die Spitze (178) aufzunehmen.

22. Vorrichtung gemäß Anspruch 21, wobei die Spitze (178) einen Durchmesser von wenigstens 0.32 cm (0.125 Zoll) aufweist.

23. Vorrichtung gemäß Anspruch 21, wobei die Spitze (178) eine kreisförmige Querschnittskonfiguration aufweist.

24. Vorrichtung gemäß den Ansprüchen 21 bis 23, wobei der erste Stempel (144) ferner eine konfigurierte Spitze (148) mit mehreren darin gebildeten länglichen Rillen (150) aufweist, und wobei die erste Form (158) mehrere Flügel (168) umfasst, wobei jeder der Flügel (168) fähig ist, mit einer der auf dem ersten Stempel (144) gebildeten Rillen (150) ineinanderzugreifen.

25. Vorrichtung gemäß Anspruch 24, wobei die auf dem ersten Stempel (144) gebildete konfigurierte Spitze (148), eine Knotenfläche (154) aufweist, die das Kräuseln der Röhre (120) erleichtert, wenn der erste Stempel (144) mit der ersten Form (158) in Kontakt gebracht wird.

26. Vorrichtung gemäß Anspruch 24, wobei die auf dem ersten Stempel (172) gebildete halbsphärisch geformte Spitze (176) eine Knotenfläche (184) beinhaltet, die das Falten und Bilden der Röhre (120) erleichtert, wenn der zweite Stempel (172) mit der zweiten Form (186) in Kontakt gebracht wird.

27. Vorrichtung gemäß Anspruch 24, wobei die erste Form (158) wenigstens vier Flügel (168) beinhaltet.

28. Vorrichtung gemäß Anspruch 24, wobei die erste Form (158) wenigstens 8 Flügel (168) beinhaltet.

29. Vorrichtung gemäß Anspruch 24, wobei der erste Stempel (144) wenigstens vier längliche Rillen (150) beinhaltet.

30. Vorrichtung gemäß den Ansprüchen 24 bis 29, wobei der erste Stempel (144) eine konfigurierte Spitze (148) mit acht darin gebildeten länglichen Rillen (150) aufweist; wobei die erste Form (158) eine Basis (166) enthält, die acht sich von dort axial nach außen erstreckende Flügel (168) aufweist, und wobei die zweite Form (186) ein Gegenstück zu dem zweiten Stempel ist, um das gekräuselte Ende der Röhre (120) in acht Falten (134) zu überführen, und um die acht Falten (134) zu einer halbsphärisch geformten Spitze (190) mit einer dahindurch gebildeten zentralen Öffnung (192) zu bilden.

31. Vorrichtung gemäß Anspruch 30, wobei der Stift (178) einen Durchmesser von Wenigstens 0.16 cm (0.062 Zoll) aufweist.

32. Vorrichtung gemäß Anspruch 30, wobei der Stift (178) einen im wesentlichen gleichbleibenden Durchmesser aufweist.

33. Vorrichtung gemäß Anspruch 30, wobei der Stift (178) ein freies Ende aufweist und, wobei der Stift sich im Durchmesser von einem größeren Durchmesser, der benachbart zu der Befestigungsstelle der Spitze liegt, zu einem kleineren Durchmesser an seinem freien Ende verjüngt.

34. Vorrichtung gemäß Anspruch 30, wobei der Stift (178) eine Länge von wenigstens 0.15 cm (0.06 Zoll) aufweist.

35. Verfahren zum Kräuseln, Falten und Bilden einer Spitze (190) auf einer Hohlröhre (120) unter Verwendung eines ersten Stempels (144, 144'), der einen röhrenförmigen Abschnitt aufweist, welcher eine derartige Größe hat, dass er in die Röhre (120) eingeführt werden kann und welcher eine konfigurierte Spitze mit mehreren darin gebildeten, länglichen Rillen aufweist und einer Schulter (156), die an einem gegenüberliegenden Ende des röhrenförmigen Abschnitts gebildet ist, welche als Haltepunkt für die Röhre (120) dient, einer ersten Form (158), die ein Gegenstück des ersten Stempels (144, 144') darstellt, und einer Röhre (120) zum Kräuseln des Endes der Röhre (120), wobei die erste Form (158) eine Basis enthält, die mehrere sich von dort axial nach außen erstreckende Flügel aufweist, wobei jeder der Flügel fähig ist, mit einer der auf dem ersten Stempel (144, 144') gebildeten Rille ineinanderzugreifen und die sich dazwischen befindende Spitze der Röhre (120) zu kräuseln, eines zweiten Stempels (172), der einen röhrenförmigen Abschnitt aufweist, der eine derartige Größe hat, dass er in die Röhre eingeführt werden kann, und welcher eine halbsphärisch geformte Spitze mit einem sich vom Scheitelpunkt der Spitze nach außen erstreckenden Stift (178) aufweist, wobei der Stift einen Durchmesser von wenigstens 1.5 mm aufweist, wobei der zweite Stempel (172) weiterhin eine am gegenüberliegenden Ende des röhrenförmigen Abschnitts gebildete Schulter (182) aufweist, welche als Haltepunkt für die Röhre (120) dient, und eine zweite Form (186), welche das Gegenstück zu dem zweiten Stempel (172) ist, aufweist, um das gekräuselte Ende der Röhre (120) in mehrere Falten (134) zu überführen und die Falten (134) in eine halbsphärisch geformte Spitze (190) zu bilden, welche eine dahindurch gebildete zentrale Öffnung (192) aufweist, wobei die Öffnung einen Durchmesser zwischen 1.5 mm und 5.0 mm aufweist, wobei die zweite Form (186) eine Basis enthält, die einen halbsphärischen Hohlraum aufweist, welcher darin einen am untersten Teil des Hohlraums gebildeten zentralen Durchgang aufweist, wobei der Hohlraum eine derartige Größe hat, um nur den Stift (178) aufzunehmen, wobei das Verfahren die folgenden Schritte umfaßt:
a) Schieben der Hohlröhre (120) auf den ersten Stempel (144, 144') bis ein Ende der Röhre (120) mit der Schulter (156) in Kontakt kommt;
b) Bewegen des ersten Stempels (144, 144') und der Röhre (120), bis sie mit der ersten Form (158) ineinandergreifen, wobei mehrere Kräusel auf dem gegenüberliegenden Ende der Röhre (120) gebildet werden;
c) Lösen des ersten Stempels (144, 144') von der ersten Form (158) und Entfernen der gekräuselten Röhre von dem ersten Stempel;
d) Schieben der Röhre (120) auf den zweiten Stempel (172), bis das nicht-gekräuselte Ende der Röhre (120) mit der Schulter (182) in Kontakt kommt;
e) Bewegen des zweiten Stempels (172) und der Röhre (120) bis sie mit der zweiten Form (186) ineinandergreifen und in Eingriff bringen des Stiftes (178) mit dem Durchgang, wobei das gekräuselte Ende der Röhre (120) in mehrere Falten (134) überführt wird und Bilden der Falten (134) zu einer halbsphärisch geformten Spitze (190), welche eine dahindurch gebildete zentrale Öffnung (192) aufweist; und
f) Lösen des zweiten Stempels von der zweiten Form und Entfernen der gefalteten und geformten Röhre (120) von dem zweiten Stempel (172).

36. Verfahren gemäß Anspruch 35, wobei, wenn ein Ende der Röhre (120) in Kontakt mit der Schulter kommt, das gegenüberliegende Ende der Röhre sich etwa auf gleicher Höhe mit der konfigurierten Spitze befindet, welche auf dem ersten Stempel (144, 144') gebildet ist.

37. Verfahren gemäß Anspruch 35, wobei der erste Stempel (144, 144') und die erste Form (158) in Eingriff gebracht werden, wobei wenigstens vier Falten in einem Ende der Röhre (120) gebildet werden.

38. Verfahren gemäß Anspruch 35, wobei der zweite Stempel (172) und die zweite Form in Eingriff gebracht werden, wobei eine Öffnung an einem Ende der Röhre (120) gebildet wird, welche eine kreisförmige Peripherie aufweist.

39. Verfahren gemäß Anspruch 35, wobei der erste Stempel (144, 144') und die erste Form (158) axial in Eingriff gebracht werden , sodass jede der auf der ersten Form gebildeten Flügel mit einer der entsprechenden auf dem ersten Stempel gebildeten Rillen axial ausgerichtet ist.

## Revendications

1. Applicateur de tampon (10) comprenant :
a) un premier élément (20) capable d'héberger un tampon absorbant (12), ledit premier élément (20) ayant un axe central longitudinal ainsi qu'une première (28) et une seconde (30) extrémités ;
b) une pointe d'insertion (32) formée d'un seul tenant avec la première extrémité (28) dudit premier élément (20) et s'étendant vers l'extérieur à partir de celui-ci, ladite pointe d'insertion (32) comprenant une pluralité de plis (50), lesdits plis (50) étant capables de se dilater vers l'extérieur tandis que ledit tampon (12) est expulsé hors dudit premier élément (20) ; et
c) un second élément (22) monté télescopique dans ladite seconde extrémité (30) dudit premier élément (20), ledit second élément (22) étant adapté à expulser ledit tampon (12) à travers ladite pointe d'insertion (32) tandis qu'il est poussé à l'intérieur dudit premier élément (20),
ledit applicateur de tampon (10) étant caractérisé en ce que ladite pointe d'insertion (32) présente une ouverture traversante (36) ayant une paroi latérale (38), ladite paroi latérale (38) étant alignée parallèlement audit axe central longitudinal, ladite ouverture ayant un diamètre compris entre 1,5 mm à 5,0 mm et ladite pluralité de plis (50) étant disposée selon une configuration hémisphérique, dans laquelle la section transversale de la configuration hémisphérique est sous-tendue par un arc (A) d'approximativement 180° et dans laquelle la configuration hémisphérique est formée avec un diamètre extérieur dimensionné de manière à être égal ou légèrement inférieur au diamètre extérieur du premier élément (20).

2. Applicateur de tampon (10) selon la revendication 1, dans lequel ledit premier élément (20) est un tube creux en papier, enroulé en spirale.

3. Applicateur de tampon (10) selon la revendication 1, dans lequel ledit premier élément (20) est un tube creux en papier convoluté.

4. Applicateur de tampon (10) selon la revendication 2, dans lequel ledit premier élément (20) est un tube creux en papier, soudé longitudinalement.

5. Applicateur de tampon (10) selon la revendication 4, dans lequel ledit tube en papier est construit à partir d'au moins un jet de matériau.

6. Applicateur de tampon (10) selon la revendication 1, dans lequel ladite pointe d'insertion (32) comprend un nombre pair de plis (50).

7. Applicateur de tampon (10) selon la revendication 1, dans lequel ladite pointe d'insertion (32) comprend un nombre impair de plis (50).

8. Applicateur de tampon (10) selon la revendication 1, dans lequel ladite pointe d'insertion (32) comprend de 3 à 32 plis (50).

9. Applicateur de tampon (10) selon la revendication 1, dans lequel ladite pointe d'insertion (32) comprend de 6 à 12 plis (50).

10. Applicateur de tampon (10) selon l'une des revendications précédentes, dans lequel ledit premier élément (20) a une surface extérieure sensiblement lisse et ladite pointe d'insertion (32) comprend au moins 3 plis (50) disposés selon une configuration hémisphérique, lesdits plis (50) étant capables de se dilater radialement vers l'extérieur tandis que ledit tampon (12) est expulsé dudit premier élément (20).

11. Applicateur de tampon (10) selon la revendication 10, dans lequel chacun desdits plis (50) a une première extrémité (52) qui coïncide avec ladite ouverture (36) et une seconde extrémité (54) qui coïncide avec un point sur une surface extérieure (26) de ladite pointe d'insertion (32).

12. Applicateur de tampon (10) selon la revendication 10, dans lequel chacun desdits plis (50) a une première extrémité (52) qui coïncide avec ladite ouverture (36) et une seconde extrémité (54) qui coïncide au niveau d'un emplacement où ladite pointe d'insertion (32) est réunie d'un seul tenant avec ledit premier élément (20).

13. Applicateur de tampon (10) selon la revendication 10, dans lequel chacun desdits plis (50) a une première extrémité (52) qui coïncide avec ladite ouverture (36) et une seconde extrémité (54) qui coïncide avec un point sur ladite surface extérieure (26) dudit premier élément (20).

14. Applicateur de tampon (10) selon la revendication 10, dans lequel ladite pointe d'insertion (32) comprend 8 plis (50) espacés les uns des autres d'une distance approximativement égale.

15. Applicateur de tampon (10) selon les revendications 1 à 6 et 8 à 14, dans lequel ledit premier élément (20) est un tube creux enroulé en spirale, ledit premier élément ayant une surface extérieure sensiblement lisse et ladite pointe d'insertion (32) comprend 8 plis (50) espacés les uns des autres d'une distance approximativement égale.

16. Applicateur de tampon selon la revendication 15, dans lequel ladite ouverture (36) a un diamètre d'environ 3,0 mm.

17. Applicateur de tampon selon la revendication 15, dans lequel ladite paroi latérale de ladite ouverture (36) contient un rayon adjacent à une surface extérieure de ladite pointe d'insertion (32).

18. Applicateur de tampon (10) selon la revendication 15, dans lequel lesdits plis (50) ont une épaisseur de moins de 0,5 mm.

19. En combinaison, un applicateur de tampon (110) et un tampon cataménial (112) ayant un nez profilé, ladite combinaison comprenant :
a) un premier élément (120) capable d'héberger ledit tampon cataménial (112), ledit premier élément (120) ayant un axe longitudinal central ainsi qu'une première (128) et une seconde (130) extrémités ;
b) une pointe d'insertion (132) formée d'un seul tenant avec ladite première extrémité (128) dudit premier élément (120) et s'étendant vers l'extérieur de celui-ci, ladite pointe d'insertion (132) comprenant une pluralité de plis (134), lesdits plis (134) étant capables de se dilater vers l'extérieur tandis que ledit tampon (112) est expulsé dudit premier élément (120) ; et
c) un second élément (122) monté télescopique dans ladite seconde extrémité (130) dudit premier élément (120), ledit second élément (122) étant adapté à expulser ledit tampon (112) à travers ladite pointe d'insertion (132) tandis qu'il est poussé à l'intérieur dudit premier élément (120) ;
ladite combinaison étant caractérisée en ce que ladite pointe d'insertion (132) dudit applicateur de tampon (110) présente une ouverture traversante (136) ayant une paroi latérale, ladite paroi latérale (38) étant alignée parallèlement audit axe central longitudinal, ladite ouverture ayant un diamètre compris entre 1,5 mm et 5,0 mm, ladite pluralité de plis (134) étant disposée pour former une configuration hémisphérique.

20. Combinaison selon la revendication 19, dans laquelle ladite pointe d'insertion (132) a une surface intérieure de forme hémisphérique et ledit nez profilé sur ledit tampon est configuré pour se conformer à ladite surface intérieure.

21. Appareil pour resserrer, plisser et former une pointe (132) sur un tube creux (120), ledit appareil comprenant :
a) un premier poinçon (144) ayant une section tubulaire (146) dimensionnée pour pouvoir être introduite à l'intérieur dudit tube (120) et un épaulement (156) formé à une extrémité opposée dudit section tubulaire (146) qui fait office de butée pour ledit tube (120) ;
b) une première matrice (158) pouvant être appariée audit premier poinçon (144) et au tube (120) pour resserrer une extrémité dudit tube (120), ladite première matrice (158) comprenant une base (166) ayant une pluralité de lamelles (168) qui se projettent axialement vers l'extérieur de celle-ci, chacune desdites lamelles (168) étant capable de venir en prise avec ledit tube (120) et de resserrer l'extrémité (132) dudit tube (120) se trouvant entre ladite matrice et ledit poinçon ;
c) un second poinçon (172) ayant une section tubulaire (174) dimensionnée de manière à pouvoir être introduite à l'intérieur dudit tube (120) et ayant une pointe hémisphérique (176) avec une goupille (178) se projetant vers l'extérieur à partir de l'apex de ladite pointe (176), ladite goupille ayant un diamètre d'au moins 1,5 mm, ledit second poinçon (172) ayant en outre un épaulement (182) formé au niveau d'une extrémité opposée de ladite section tubulaire (174) qui fait office de butée pour ledit tube (120) ; et
d) une seconde matrice (186) pouvant s'apparier audit second poinçon (172) pour conformer ladite extrémité resserrée dudit tube (120) en une pluralité de plis (134) et conformer lesdits plis (134) en une pointe hémisphérique (190) ayant une ouverture centrale traversante (192), ladite seconde matrice (186) comprenant une base (194) ayant une cavité hémisphérique (200) formée dans celle-ci avec un passage central (202) formé au fond de ladite cavité (200), ladite cavité (200) étant dimensionnée pour recevoir à la fois ledit second poinçon (172) et ledit tube (120), et ledit passage (202) étant dimensionné pour ne recevoir que ladite goupille (178).

22. Appareil selon la revendication 21, dans lequel ladite goupille (178) a un diamètre d'au moins 0,32 cm (0,125 pouce).

23. Appareil selon la revendication 21, dans lequel ladite goupille (178) a, en coupe transversale, une configuration circulaire.

24. Appareil selon les revendications 21 à 23, dans lequel ledit premier poinçon (144) a en outre une pointe configurée (148) avec une pluralité de rainures allongées (150) formées dans celle-ci, et dans lequel ladite première matrice (158) comprend une pluralité de lamelles (168), chacune desdites lamelles (168) étant capable de venir en prise avec l'une desdites rainures (150) formées sur ledit premier poinçon (144).

25. Appareil selon la revendication 24, dans lequel ladite pointe configurée (148) formée sur ledit premier poinçon (144) comprend une zone moletée (154) qui facilite le resserrage dudit tube (120) lorsque ledit premier poinçon (144) est amené en contact avec ladite première matrice (158).

26. Appareil selon la revendication 24, dans lequel ladite pointe hémisphérique (176) formée sur ledit second poinçon (172) comprend une zone moletée (184) qui facilite le plissage et la formation dudit tube (120) lorsque ledit second poinçon (172) est amené en contact avec ladite seconde matrice (186).

27. Appareil selon la revendication 24, dans lequel ladite première matrice (158) comprend au moins 4 lamelles (168).

28. Appareil selon la revendication 24, dans lequel ladite première matrice (158) comprend au moins 8 lamelles (168).

29. Appareil selon la revendication 24, dans lequel ledit premier poinçon (144) comprend au moins 4 rainures allongées (150).

30. Appareil selon les revendications 24 à 29, dans lequel ledit premier poinçon (144) a une pointe configurée (148) avec 8 rainures allongées (150) formées sur celle-ci, dans lequel ladite première matrice (158) comprend une base (166) ayant 8 lamelles (168) se projetant axialement vers l'extérieur de celle-ci, et dans lequel ladite seconde matrice (186) peut être appariée audit second poinçon (172) pour transformer ladite extrémité resserrée dudit tube (120) en 8 plis (134) et pour conformer lesdits 8 plis (134) en une pointe hémisphérique (190) ayant une ouverture centrale traversante (192).

31. Appareil selon la revendication 30, dans lequel ladite goupille (178) a un diamètre d'au moins 0,16 cm (0,062 pouce).

32. Appareil selon la revendication 30, dans lequel ladite goupille (178) a un diamètre essentiellement constant.

33. Appareil selon la 'revendication 30, dans lequel ladite goupille (178) a une extrémité libre et ladite goupille va en s'effilant depuis un plus grand diamètre à proximité de son point de fixation sur ladite pointe vers un plus petit diamètre au niveau de son extrémité libre.

34. Appareil selon la revendication 30, dans lequel ladite goupille (178) a une longueur d'au moins 0,15 cm (0,06 pouce).

35. Procédé pour resserrer, plisser et former une pointe (190) sur un tube creux (120) en utilisant un premier poinçon (144, 144') ayant une section tubulaire dimensionnée pour pouvoir être introduite à l'intérieur dudit tube (120) et ayant une pointe configurée avec une pluralité de rainures allongées formées sur celle-ci, et un épaulement (156) formé au niveau d'une extrémité opposée de ladite section tubulaire qui fait office de butée pour ledit tube (120), une première matrice (158) pouvant être appariée audit premier poinçon (144, 144') et au tube (120) pour resserrer une extrémité dudit tube (120), ladite première matrice (158) comprenant une base ayant une pluralité de lamelles se projetant axialement vers l'extérieur depuis celle-ci, chacune desdites lamelles étant capable de venir en prise avec l'une desdites rainures formées sur ledit premier poinçon (144, 144') et de resserrer la pointe dudit tube (120) positionné entre ladite matrice et ledit poinçon, un second poinçon (172) ayant une section tubulaire dimensionnée de manière à pouvoir être introduite à l'intérieur dudit tube et ayant une pointe hémisphérique avec une goupille (178) se projetant vers l'extérieur à partir de l'apex de ladite pointe, ladite goupille ayant un diamètre d'au moins 1,5 mm, ledit second poinçon (172) ayant en outre un épaulement (182) formé au niveau d'une extrémité opposée de ladite section tubulaire qui fait office de butée pour ledit tube (120) et une seconde matrice (186) pouvant être appariée audit second poinçon (172) pour transformer ladite extrémité resserrée dudit tube (120) en une pluralité de plis (134) et conformer lesdits plis (134) en une pointe hémisphérique (190) ayant une ouverture centrale traversante (192), ladite ouverture ayant un diamètre compris entre 1,5 mm et 5,0 mm, ladite seconde matrice (186) comprenant une base dans laquelle est formée une cavité hémisphérique avec un passage central formé dans le fond de ladite cavité, ladite cavité étant dimensionnée pour ne recevoir que ladite goupille (178), ledit procédé comprenant les étapes qui consistent à :
a) faire coulisser ledit tube creux (120) sur ledit premier poinçon (144, 144') jusqu'à ce qu'une extrémité dudit tube (120) entre en contact avec ledit épaulement (156) ;
b) déplacer ledit premier poinçon (144, 144') et le tube (120) pour qu'ils viennent en prise avec ladite première matrice (158) pour former une pluralité de resserrements au niveau d'une extrémité opposée dudit tube (120) ;
c) désengager ledit premier poinçon (144, 144') de ladite première matrice (158) et retirer ledit tube resserré dudit premier poinçon ;
d) faire coulisser ledit tube (120) sur ledit second poinçon (172) jusqu'à ce que l'extrémité non resserrée dudit tube (120) entre en contact avec ledit épaulement (182) ;
e) déplacer ledit second poinçon (172) et le tube (120) pour qu'ils viennent en prise avec ladite seconde matrice (186) et engager ladite goupille (178) dans ledit passage pour transformer ladite extrémité resserrée dudit tube (120) en une pluralité de plis (134) et conformer lesdits plis (134) en une pointe hémisphérique (190) ayant une ouverture centrale traversante (192) ; et
f) désengager ledit second poinçon de ladite seconde matrice et retirer ledit tube plissé et conformé (120) dudit second poinçon (172).

36. Procédé selon la revendication 35, dans lequel, lorsqu'une extrémité dudit tube (120) entre en contact avec ledit épaulement, l'extrémité opposée dudit tube est approximativement au niveau de ladite pointe configurée formée sur ledit premier poinçon (144, 144').

37. Procédé selon la revendication 35, dans lequel ledit premier poinçon (144, 144') et ladite première matrice (158) viennent en prise pour former au moins 4 plis sur une extrémité dudit tube (120).

38. Procédé selon la revendication 35, dans lequel ledit second poinçon (172) et ladite seconde matrice viennent en prise pour former une ouverture au niveau d'une extrémité dudit tube (120) ayant une périphérie circulaire.

39. Procédé selon la revendication 35, dans lequel ledit premier poinçon (144, 144') et ladite première matrice (158) viennent en prise axialement de telle sorte que chacune desdites lamelles formées sur ladite première matrice est alignée axialement avec l'une desdites rainures correspondantes formées sur ledit premier poinçon.
